# EUROPEAN PATENT SPECIFICATION

(11) **EP 4 562 185 B1**
(45) Date of publication and mention of the grant of the patent: **15.04.2026**
(21) Application number: 23764729.2
(22) Date of filing: 21.07.2023
(51) Int. Cl.: C12Q 1/6886

(54) **METHOD FOR PREDICTING THE DEVELOPMENT OF RESISTANCE TO BRAF INHIBITING DRUGS, ALONE OR IN COMBINATION WITH MEK INHIBITING DRUGS, IN AN ANTI-TUMOUR TREATMENT**
VERFAHREN ZUR VORHERSAGE EINER RESISTENZENTWICKLUNG GEGEN BRAF-HEMMENDE ARZNEIMITTEL, ALLEIN ODER IN KOMBINATION MIT MEK-HEMMENDEN ARZNEIMITTELN, BEI EINER ANTITUMORBEHANDLUNG
PROCÉDÉ DE PRÉDICTION DU DÉVELOPPEMENT DE LA RÉSISTANCE AUX MÉDICAMENTS INHIBITEURS DE BRAF, SEULS OU EN COMBINAISON AVEC DES MÉDICAMENTS INHIBITEURS DE MEK, DANS UN TRAITEMENT ANTI-TUMORAL

(30) Priority: 25.07.2022 IT 202200015630
(43) Date of publication of application: 04.06.2025
(73) Proprietor: Istituti Fisioterapici Ospitalieri (IFO), 00144 Roma (RM) (IT); Istituto Nazionale Tumori IRCCS - Fondazione G. Pascale, 80131 Napoli (NA) (IT); Sapienza Universita di Roma, 00185 Rome (IT)
(72) Inventor: CILIBERTO, Gennaro, 00144 Roma (RM) (IT); FATTORE, Luigi, 00144 Roma (RM) (IT); TERRENATO, Irene, 00144 Roma (RM) (IT); MANCINI, Rita, 00185 Roma (RM) (IT); ASCIERTO, Paolo Antonio, 80131 Napoli (NA) (IT); RUGGIERO, Ciro Francesco, 80131 Napoli (NA) (IT)
(74) Representative: Gitto, Serena
(86) International application number: PCT/IT2023/050171
(87) International publication number: WO 2024/023860

(56) References cited:
- WO-A1-2019/198115
- FATTORE LUIGI ET AL: "Reprogramming miRNAs global expression orchestrates development of drug resistance in BRAF mutated melanoma", CELL DEATH & DIFFERENTIATION, NATURE PUBLISHING GROUP, GB, vol. 26, no. 7, 25 September 2018 (2018-09-25), pages 1267 - 1282, XP036797465, ISSN: 1350-9047, [retrieved on 20180925], DOI: 10.1038/S41418-018-0205-5
- ANONYMOUS: "Immunotherapy Bridge 2020 and Melanoma Bridge 2020: meeting abstracts", vol. 19, no. S1, 24 March 2021 (2021-03-24), XP093023966, Retrieved from the Internet <URL:http://link.springer.com/article/10.1186/s12967-020-02685-2/fulltext.html> DOI: 10.1186/s12967-020-02685-2
- RUGGIERO CIRO F ET AL: "Circulating levels of miR-204-5p predict response to BRAF and MEK inhibitors in metastatic melanoma patients", 2020, XP093023471, Retrieved from the Internet <URL:https://www.melanomaimi.it/images/CONGRESSO_NAZ_2020/P502.pdf> [retrieved on 20230214]
- MOTTI MARIA LETIZIA ET AL: "MicroRNAs as Key Players in Melanoma Cell Resistance to MAPK and Immune Checkpoint Inhibitors", INTERNATIONAL JOURNAL OF MOLECULAR SCIENCES, vol. 21, no. 12, 26 June 2020 (2020-06-26), pages 4544, XP093023310, DOI: 10.3390/ijms21124544
- RUGGIERO CIRO FRANCESCO ET AL: "Identification of a miRNA-based non-invasive predictive biomarker of response to target therapy in BRAF-mutant melanoma", vol. 12, no. 17, 24 October 2022 (2022-10-24), AU, pages 7420 - 7430, XP093023313, ISSN: 1838-7640, Retrieved from the Internet <URL:https://www.ncbi.nlm.nih.gov/pmc/articles/PMC9691363/pdf/thnov12p7420.pdf> DOI: 10.7150/thno.77761

## Description

The present invention concerns a method for predicting the development of resistance to BRAF inhibiting drugs, alone or in combination with MEK inhibiting drugs (i.e., MAPK pathway inhibitors or simply MAPKi), in anti-tumour treatment. In particular, the present invention concerns a method for predicting the development of resistance to BRAF and/or MEK inhibiting drugs in anti-tumour treatment, wherein said method comprises measuring the expression levels of the two microRNAs miR-579-3p and miR-4488 in a biological sample collected from a cancer patient before starting the anti-tumour treatment with BRAF and/or MEK inhibitors.

It is known that some tumors can be treated with MAPK pathway inhibitors.

In particular, the treatment of metastatic melanoma has seen considerable progress in recent years thanks to advent of targeted therapy with MAPKi and of immunotherapy with the immuno-checkpoint inhibitors [1,2]. For target therapy the gold standard consists in the combinatorial use of a BRAF and a MEK inhibitor directed to block the BRAF/MEK/MAPK signaling pathway in BRAFV600 mutated melanomas (MAPKi). Although this approach provides significant benefit in terms of objective responses, time to progression and overall survival, the effects are often vanished by innate or acquired drug resistance which results in tumor relapses with a median time-to-recurrence of approximately 15 months [2,3]. Relapsing tumors are highly aggressive and hardly treatable [4,5]. Of note, although immunotherapy is often utilized as a salvage option after disease progression to MAPKi in BRAF-mutated melanoma patients, important studies have shown that acquired resistance to MAPKi is a negative factor for subsequent response to immunotherapy [9].

Based on these important evidences it is necessary to develop new diagnostic tools able to predict which therapy might offer the best benefits in terms of response in advanced melanoma patients.

Nowadays tissue biopsy is the "gold standard" test for cancer diagnosis. However, the removal of metastatic tumor tissue by surgery is an invasive process and it is associated with many limitations [10-12]. Moreover, this kind of procedure is incompatible for clinical longitudinal monitoring in melanoma patients. In contrast, liquid biopsies directed to analyze biomarkers released by the tumor in the blood offer a great opportunity to overcome these limitations. This approach allows to monitor longitudinally patient's response to a specific treatment and decide whether to continue the therapy or promptly change it. [12,13]. Several evidences over the last years have highlighted the potential use of circulating molecular biomarkers for melanoma diagnosis and prognosis. In fact, this approach exploits several biomarkers present in the blood including circulating tumor cells (CTCs), cell-free circulating tumor DNA (ctDNA), lactate dehydrogenase (LDH), S100 calcium-binding protein B (S100B) and the cell-free circulating RNA (cfRNA) suitable as prognostic factors for monitoring disease progression in advanced melanoma patients [10,14-19].

Among the latters a growing relevance has been attributed to a class of small non-coding RNA called microRNAs (miRNAs). Inside the cells these small molecules are essential regulators of the post-transcriptional gene expression and their altered balance is associated with several pathological conditions including cancer [14,20-24]. Moreover, the alteration of miRNAs is also correlated with the establishment of resistance to therapy [25,26,27]. In addition to the canonical intracellular functions, several findings demonstrated that miRNAs can be released and travel in the biological fluids protected by proteins such as AGO2 or in alternative they can be encapsulated with high stability into extracellular vesicles (EVs) such as the exosomes [28,29,30]. Of note miRNAs expression can be easily detected through the standard real-time quantitative reverse transcription polymerase chain reaction (qRT-PCR).

For these reasons, circulating miRNAs are emerging more and more as potential non-invasive biomarkers to diagnose the development of many cancers such as breast, leukemia, lung cancer, pancreatic cancer and also melanoma [31-36]. For instance, in a study it has been demonstrated that serum-exosomal miR-105 may have the potential to predict, or diagnose at an early stage, patients who may develop or have already developed breast cancer metastasis [37]. Again, another study in ductal adenocarcinoma (PDAC) from blood plasma has brought to light the potential role of miRNA-483-3p as biomarkers in PDAC. In particular, it was observed that the deregulation of this miRNA is an early event of PDAC. Indeed miR-483-3p is overexpressed in serum and serum exosomes of PDAC patients suggesting that is could be a potential liquid biopsy biomarker for early detection of PDAC [38]. Finally, also in lung cancer several studies have highlighted the diagnostic and/or prognostic potential role of miRNAs carried out by the exosomes. Recently, it was reported that an exosomal miRNA profile involving let-7 and other miRNAs able to drastically discriminate lung cancer patients from healthy controls [39].

Also in melanoma several works have documented that distinct sets of miRNAs could be utilized as prognostic biomarkers for cancer development or predict the outcomes of treatment responses [34,35,36]. For example, Margue et al. [23, 40] demonstrated that miR-301a-3p is down-regulated in stage III/IV melanoma patients relative to healthy controls. Again Greenberg et al. [23, 41] reported miR-29c-5p and miR-324-3p to be lower in the serum of metastatic melanoma patients (stage IV) compared to healthy control individuals.

It is important pointing out there are two important issues regarding circulating miRNAs, i.e. their normalization and the quantification in plasma/serum. The first problem arises from the observation that there are no miRNAs that can act as a circulating reference. Unfortunately, the canonical reference miRNAs used in tissue samples, such as RNU48 and RNU6, are not suitable for normalizing extracellular miRNAs due their degradation by RNase-mediated in the bloodstream [23]. Also total RNA extracted from serum/plasma samples is usually below the threshold of standard quantification methods such as UV spectrophotometry or fluorescence-based spectrophotometry.

To overcome both the above-mentioned issues a fixed volume of plasma or serum is often chosen as the input for the reverse transcription reaction (RT). Other approaches to normalize miRNA expression are Global Mean (GMN) and REfFinder (RF) methods. Global mean normalization consists in determining Ct values for the number of miRNAs tested per each sample. High Ct values (above certain threshold) will have to be removed (noise) and the remaining Ct values should be used to calculate mean Ct across all miRNAs per each sample separately. The arithmetic average Ct value is then calculated for each individual sample and subsequently subtracted from each individual Ct value for that sample. RefFinder is a web-based comprehensive tool developed for evaluating and screening reference genes from extensive experimental datasets. In detail RefFinder performs a quick analysis based on the currently available major computational programs (geNorm, Normfinder, BestKeeper, and the comparative Delta-Ct method) to compare and rank the tested candidate reference genes. In this way it is possible to establish which miRNA undergoes less fluctuations between the various methods analyzed by the program and therefore can act as a reference miRNA.

During last years, the role of miRNAs in melanoma progression and resistance to therapy has been studied [26,45,46,47]. In a first study, a novel oncosuppressive miRNA, namely miR-579-3p, was identified, which was down-regulated in BRAF-mutant melanoma cells and even more when they develop resistance to MAPKi. Furthermore, this miRNA is strongly down-regulated also in matched tumor samples from patients before and after the development of resistance to targeted therapies [45]. This first study was followed by a comprehensive analysis of the changes of the whole miRnome deregulated during development of resistance to MAPKi *in vitro.* This led to the identification of more than 20 deregulated miRNAs and to the in-depth characterization of five of them [46]. In particular, three miRNAs (miR-9-5p, miR-4443 and miR-4488) resulted strongly up-regulated whereas other two miRNAs were found downregulated (miR-199b-5p and miR-204-5p) [47,48] during the development of drug resistance. These miRNAs were found significantly modulated also in solid and liquid biopsies of melanoma patients after disease recurrence [46]. In the specific case of liquid biopsies, it was found that miR-199b-5p expression levels were downregulated in the plasma of 25 melanoma patients post-MAPKi treatment as compared to the plasma from untreated patients. On the opposite, miR4488 levels were significantly increased in patients after MAPKi treatment [46].

Patent application WO 2019/198115 is also known, which concerns a method for in vitro diagnosis of resistance to MAPK pathway inhibiting drugs in tumors, wherein two or more of miRNAs among miR-199b-5p, miR-204-5p, miR-4443 and miR-4488 are detected in a biological sample by measuring their expression during the therapy. According to this patent application, the above-mentioned miRNAs are predictive of drug resistance during the treatment with MAPK pathway inhibiting drugs.

So far known methods are not able to predict resistance to MAPK pathway inhibiting drugs before starting the treatment with MAPKi. Important disadvantages are connected with a late detection of said resistance, since the patient would suffer from side effects due to the therapy without receiving any benefit. In addition, a late detection of said resistance does not allow an early assignment of an alternative treatment such as immunotherapy with checkpoint inhibitors (ICI) for a patient who is resistant to MAPK pathway inhibiting drugs, thus decreasing the chances of recovering. All these disadvantages are also connected to higher medical costs.

In the light of the above, it is therefore apparent the need to provide new diagnostic methods and biomarkers for predicting drug resistance in cancer patients, in particular melanoma patients, able to overcome the disadvantages of known methods.

According to the present invention has now been found that miR-579-3p and miR-4488 are, together, a predictive biomarker able to discriminate advanced melanoma patients who benefited from MAPKi treatments. In particular, as shown in the experimental data reported below, a restrospective analysis of 70 serum samples derived from BRAF-mutated melanoma patients treated with MAPKi therapy was carried out in first instance and 6 circulating miRNAs were evaluated in detail as predictors of response to therapy before starting treatments, three oncomiRs (miR-9-5p, miR-4443 and miR-4488) and three oncosuppressors (miR-579-3p, miR-204-5p and miR-199b-5p). Results showed that among the candidates, miR-579-3p and miR-4488 resulted to be the only promising predictive biomarker able to discriminate advanced melanoma patients who benefited from MAPKi treatments.

The above-mentioned two miRNAs are able to predict the response to MAPKi therapy before starting the therapeutic treatment.

In addition, according to the present invention it has been found that the expression ratio of miR-4488:miR-579-3p shows a good sensitivity and specificity for predicting disease progression when the patient is treated with MAPKi.

Therefore, according to the present invention, for the first time, a miRNA-based assay as predictor of therapy response to MAPKi therapy, before starting said therapy, is provided.

It is therefore specific object of the present invention a method *in vitro* for identifying a non-responsive tumour patient to an anti-tumour treatment with a BRAF inhibitor alone or in combination with a MEK inhibitor (MAPK pathway inhibiting drugs), said method comprising
measuring, or obtaining a measurement of, the expression of both microRNAs miR-579-3p (MIMAT0003244) and miR-4488 (MIMAT0019022) in a biological sample of a tumour patient before starting said anti-tumour treatment,
calculating or obtaining the ratio between the expression of miR-4488 and the expression of miR-579-3p (miR-4488:miR-579-3p),
wherein
said tumour patient is a non-responsive tumour patient when said ratio is higher than the ratio between the expression of miR-4488 and the expression of miR-579-3p measured in a biological sample of a responsive tumour patient to the same anti-tumour treatment with a BRAF inhibitor alone or in combination with a MEK inhibitor before starting said anti-tumour treatment.

For non-responsive tumour patient is intended a tumour patient undergoing progressive disease (PD) within 6 months from the start of a treatment with a BRAF inhibitor either alone or in combination with a MEK inhibitor.

For responsive tumour patient is intended a tumour patient who is experiencing a partial or complete response (PR OR CR) or a stable disease (SD), such as according to the RECIST criteria, for at least six months after the start of a treatment with a BRAF inhibitor either alone or in combination with a MEK inhibitor.

According to the present invention, identifying a non-responsive tumour patient is intended as identifying a tumour patient who has a high probability of developing a progressive disease upon therapy with BRAF inhibitor either alone or in combination with a MEK inhibitor.

Said ratio between the expression of miR-4488 and the expression of miR-579-3p measured, before starting said anti-tumour treatment, in a biological sample of a responsive tumour patient can be the average value or the median value of the ratios calculated in a group of responsive tumour patients.

For the expression of microRNAs miR-579-3p and miR-4488 is intended the quantity of the expressed miRNA.

According to the method of the present invention, when said tumour patient is a non-responsive tumour patient, said ratio can be higher than a cut-off ratio value obtained by the following steps:
- measuring, or obtaining a measurement of, the expression of both microRNAs miR-579-3p and miR-4488 in biological samples of a population of tumour patients before starting an anti-tumour treatment with a BRAF inhibitor alone or in combination with a MEK inhibitor, wherein said population of tumour patients comprises a group of non-responsive tumor patients and a group of responsive tumour patients;
- calculating the ratio between the expression of miR-4488 and the expression of miR-579-3p for each tumour patient (miR-4488:miR-579-3p);
- obtaining a cut-off ratio value through a ROC curve for distinguishing non-responsive tumour patients from responsive tumour patients.

According to the present invention, when said tumour patient is a non-responsive tumour patient
the expression of miR-579-3p measured in the biological sample of said tumour patient can be lower than the expression of miR-579-3p measured in a biological sample of a responsive tumour patient to said anti-tumour treatment with a BRAF inhibitor alone or in combination with a MEK inhibitor before starting said anti-tumour treatment
   and
the expression of miR-4488 measured in the biological sample of said tumour patient can be higher than the expression of miR-4488 measured in a biological sample of a responsive tumour patient to said anti-tumour treatment with a BRAF inhibitor alone or in combination with a MEK inhibitor before starting the anti-tumour treatment.

According to an embodiment of the present invention, the measured expression of miR-579-3p and the measured expression of miR-4488 can be determined as absolute levels (or absolute values) without a normalization relative to the expression of other miRNAs.

According to a further embodiment of the present invention, the measured expression of miR-579-3p and the measured expression of miR-4488 can be normalized to the expression of miR-579-3p, miR-4488, miR-204-5p (MIMAT0000265), miR-199b-5p (MIMAT0000263), miR-9-5p (MIMAT0000441) and miR-4443 (MIMAT0018961).In particular, according to the present invention, when the expression of the miRNAs is measured by PCR and a Ct value is obtained as a measure of the expression, a normalized expression can be calculated as follows: a mean value of the Ct values is calculated from the measured expression of each of the above mentioned six miRNAs; then, the mean value is subtracted from the single Ct value measured for each miRNA, obtaining a ΔCt value for each miRNA; then, a normalized expression value can be calculated for each miRNA by applying the formula 2^{-ΔCt}.

According to a further embodiment of the present invention, the measured expression of miR-579-3p and the measured expression of miR-4488 can be normalized to the expression of miR-199b-5p.

According to the method of the present invention, therefore, the expression of said microRNAs can be measured by using pairs of primers and/or probes suitable for the purpose. It is known that primers and probes are oligonucleotide sequences complementary to the microRNA sequences to be detected. According to the present invention, said method can be carried out by using one or more synthetic sequences complementary to at least a part of miR-579-3p, one or more synthetic sequences complementary to at least a part of miR-4488. In particular, said one or more synthetic sequences can be primers and/or probes.

According to the present invention, the expression of the miRNAs can be measured by means of Real Time PCR, Droplet Digital PCR, Microarray, RNA hybridization methods, such as Northern Blot or Dot Blot, RNA Next Generation Sequencing, preferably Real Time PCR.

According to the present invention, the BRAF inhibitor can be selected among Vemurafenib, Dabrafenib, Encorafenib (BRAF inhibitors) and the MEK inhibitor is selected among and Cobimetinib, Trametinib, Binimetinib (MEK inhibitors).

According to the present invention, the tumour patient can a patient suffering from a BRAF-mutated tumor.

According to the present invention, the tumour patient can be a patient suffering from a tumour chosen from the group consisting of melanoma, Colorectal cancer, papillary thyroid carcinoma, non-small cell lung cancer, brain tumour, non-Hodgkin lymphoma.

According to the experimental results described in the example, it is plausible for a person skilled in the art that the method of the present invention is effective for any tumour treated with BRAF/MEK pathway inhibiting drugs that can become resistant to said BRAF/MEK pathway inhibiting drugs. In fact, the resistance depends on genetic alterations that cause reactivation of MAPK signalling independently from the kind of tumour. Therefore, the fact that a tumour patient non-responsive to the anti-tumour treatment with a BRAF inhibitor and/or a MEK inhibitor presents an expression of miR-579-3p lower than the expression of miR-579-3p in a responsive patient and the expression of miR-4488 higher than the expression of miR-4488 in a responsive patient depend directly on said genetic alterations and not on the kind of tumour.

According to the method of the present invention, the biological sample can be a liquid biological sample such as blood, serum, plasma, urine.

The present invention concerns also a method of diagnosing and treating a non-responsive tumour patient to an anti-tumour treatment with a BRAF inhibitor alone or in combination with a MEK inhibitor (MAPK pathway inhibiting drugs), said method comprising:
a) obtaining a measurement of the expression of both microRNAs miR-579-3p (MIMAT0003244) and miR-4488 (MIMAT0019022) in a biological sample of a tumour patient before starting said anti-tumour treatment,
b) calculating the ratio between the expression of miR-4488 and the expression of miR-579-3p (miR-4488:miR-579-3p);
c) identifying a non-responsive tumour patient to an anti-tumour treatment with a BRAF inhibitor alone or in combination with a MEK inhibitor, wherein in said non-responsive tumour patient said ratio is higher than the ratio between the expression of miR-4488 and the expression of miR-579-3p measured in a biological sample of a responsive tumour patient to the same anti-tumour treatment with a BRAF inhibitor alone or in combination with a MEK inhibitor before starting said anti-tumour treatment,
   and
d) treating the patient with immunotherapy instead of a BRAF inhibitor and/or a MEK inhibitor or with an antagonist of at least one of miR-4443 and miR-4488 and/or a miRNA mimic of at least one of miR-199b-5p, miR-204-5p and miR-579-3p, and a BRAF inhibitor, alone or in combination with a MEK inhibitor,
wherein said antagonist can be selected from the group consisting of Locked Nucleic Acid targeting miR-4443, Locked Nucleic Acid targeting miR-4488, antimiR-4443: aaaacccacgcctccaa (SEQ ID NO:1), and antimiR-4488: cgccggagcccgccccct (SEQ ID NO:2), and
wherein said miRNA mimic can be selected from the group consisting of miR-199b-5p mimic: cccaguguuuagacuaucuguuc (SEQ ID NO:3), miR-204-5p mimic: uucccuuugucauccuaugccu (SEQ ID NO:4) and miR-579-3p mimic: uucauuugguauaaaccgcgauu (SEQ ID NO:5).

The present invention concerns also a method for obtaining a cut-off ratio value for distinguishing non-responsive tumour patients to an anti-tumour treatment with a BRAF inhibitor alone or in combination with a MEK inhibitor from responsive tumour patients to the same treatment, said method comprising
- measuring, or obtaining a measurement of, the expression of both microRNAs miR-579-3p and miR-4488 in biological samples of a population of tumour patients before starting an anti-tumour treatment with a BRAF inhibitor alone or in combination with a MEK inhibitor, wherein said population of tumour patients comprises a group of non-responsive tumour patients and a group of responsive tumour patients;
- calculating the ratio between the expression of miR-4488 and the expression of miR-579-3p for each tumour patient (miR-4488:miR-579-3p);
- obtaining a cut-off ratio value through a ROC curve.

The present invention now will be described by an illustrative, but not limitative way, according to preferred embodiments thereof, with particular reference to the examples and the enclosed drawings, wherein:
- **Figure 1** shows an on overview of the whole project. In detail, total RNAs were extracted from 70 serum samples of BRAF-mutated melanoma patients and then processed by qRT-PCR to assess the levels of 6 chosen miRNA candidates. Normalization of results have been performed through different methods and used to plot ROC and Kaplan-Meier curves. miR-4488/miR-579-3p (miRatio) showed the best predictive value.
- **Figure 2** is a box plot graph showing that circulating levels (2^-dCt values calculated through GM normalization) of miR-4488 resulted significantly up-regulated in patients who did not benefit of treatments (PD) as compared to patients who benefited from MAPKi (DC).
- **Figure 3** is a box plot graph showing that circulating levels (2^-dCt values calculated through GM normalization) of miR-579-3p resulted significantly down-regulated in patients who did not benefit from treatment (PD) as compared to patients who benefited from MAPKi (DC).
- **Figure 4** is a box plot graph showing that expression levels (2^-dCt values calculated through GM normalization) of miR-4443 resulted not significantly able to distinguish PD vs DC patients.
- **Figure 5** is a box plot graph showing that expression levels (2^-dCt values calculated through GM normalization) of miR-199b-5p resulted not significantly able to distinguish PD vs DC patients.
- **Figure 6** is a box plot graph showing that expression levels (2^-dCt values calculated through GM normalization) of miR-9-5p resulted not significantly able to distinguish PD vs DC patients.
- **Figure 7** is a box plot graph showing that expression levels (2^-dCt values calculated through GMN normalization) of miR-204-5p resulted not significantly able to distinguish PD vs DC patients
- **Figure 8** shows that miR-4488 has the highest expression levels in basal serum samples from patients who did not benefit from MAPKi treatments. Melanoma patients were subdivided according to RECIST criteria (CR = complete response; PR = partial response; SD = stable disease; PD = progressive disease).
- **Figure 9** shows that miR-579-3p expression levels progressively decrease from CR to PD patients. Melanoma patients were subdivided according to RECIST criteria (CR = complete response; PR = partial response; SD = stable disease; PD = progressive disease).
- **Figure 10** shows that a significant negative Spearman correlation (r=0.4114, P <0.05) was observed in the circulating levels of miR-4488 vs miR-579-3p in basal samples.
- **Figure 11** is a box plot graph showing that the relative ratio of the circulating levels of miR-4488 vs miR-579-3p (miRatio) resulted significantly up-regulated in patients who did not benefit of treatments (PD) as compared to patients who benefited from MAPKi (DC).
- **Figure 12** shows that miRatio has the highest expression levels in basal serum samples from patients who did not benefit of MAPKi treatments. Melanoma patients were subdivided according to RECIST criteria (CR = complete response; PR = partial response; SD = stable disease; PD = progressive disease).
- **Figure 13** shows receiver operating characteristic (ROC) curves estimating the predictive value of miR-4488, miR-579-3p and miRatio (2^-dCt values calculated through GM normalization) as markers of drug resistance in basal serum samples.
- **Figure 14** shows Kaplan-Meier curves showing that higher basal levels of miR-579-3p (GM normalization) predict better response to therapy in terms of PFS months (Log-rank p=0.0045).
- **Figure 15** shows Kaplan-Meier curves showing that higher basal levels of miR-4488 (GM normalization) predict worst response to therapy in terms of PFS months (Log-rank p=0.0370).
- **Fig.16** shows Kaplan-Meier curves showing that higher basal levels of miRatio (GM normalization) predict worst response to therapy in terms of PFS months (Log-rank p=0.0024).
- **Figure 17** shows receiver operating characteristic (ROC) curves estimating the predictive value of miR-4488, miR-579-3p and miRatio (2^-dCt values obtained through GM normalization) as markers of OS in basal serum samples.
- **Figure 18** shows Kaplan-Meier curves showing that basal levels of miR-579-3p (GM normalization) are not predictive of OS (Log-rank p=NS).
- **Figure 19** shows Kaplan-Meier curves showing that basal levels of miR-4488 (GM normalization) are not predictive of OS (Log-rank p=NS).
- **Figure 20** Kaplan-Meier curves showing that basal levels of miRatio (GM normalization) are not predictive of OS (Log-rank p=NS).
- **Figure 21** shows a heat map summarizing RefFinder normalization result from the less stable (i.e. miR-4488) to the most stable (i.e. miR-199b-5p).
- **Figure 22** shows receiver operating characteristic (ROC) curves estimating the predictive value of miR-4488, miR-579-3p and miRatio (2^-dCt values obtained through RF normalization) as markers of drug resistance in basal serum samples.
- **Figure 23** shows Kaplan-Meier curves showing that higher basal levels of miRatio (RF normalization) predict worst response to therapy in terms of PFS months (Log-rank p=0.0071).
- **Figure 24** shows receiver operating characteristic (ROC) curves estimating the predictive value of miR-4488, miR-579-3p and miRatio (absolute Ct values) as markers of drug resistance in basal serum samples.
- **Figure 25** Kaplan-Meier curves showing that higher basal levels of miR-579-3p (absolute Ct values) predict better response to therapy in terms of PFS months (Log-rank p=0.0007).
- **Figure 26** shows Kaplan-Meier curves showing that higher basal levels of miR-4488 (absolute Ct values) predict worst response to therapy in terms of PFS months (Log-rank p=0.0782).
- **Figure 27** shows Kaplan-Meier curves showing that higher basal levels of miRatio (absolute Ct values) predict worst response to therapy in terms of PFS months (Log-rank p=0.0019).
- **Figure 28** is a bar graph showing that patients with Ct values of miR-579-3p under cut-off (high expression) have longer PFS as compared to patients with Ct values over cut-off (low expression).
- **Figure 29** is a bar graph showing that patients with Ct values of miR-4488 under cut-off (high expression) have shorter PFS as compared to patients with Ct values over cut-off (low expression).
- **Figure 30** is a forest plot showing that the circulating levels of miRNA candidates miR-579-3p and miR-4488 are predictive of PFS differently from LDH (univariate analysis). Considering the multivariate subgroup of analysis, the combination of LDH with circulating miRNAs did not produce any improvement for predicting PFS. Hazard Ratio value (HR) > 1 is a risk factor in PFS. HR < 1 is a protective factor of PFS.
- **Figure 31** is a forest plot showing that the circulating levels of the miRNA candidates are not predictive of OS differently from LDH levels (univariate analyses). Considering the multivariate subgroup of analysis, the combination of LDH with circulating miRNAs did not produce any improvement for predicting OS. Hazard Ratio value (HR) > 1 is a risk factor in OS. HR < 1 is a protective factor of OS.
- **Figure 32** shows receiver operating characteristic (ROC) curves estimating the predictive value of LDH levels as predictive markers of drug resistance in basal serum samples.
- **Figure 33** shows receiver operating characteristic (ROC) curves estimating the predictive value of LDH levels as predictive markers of OS in basal serum samples.
- **Figure 34** shows Kaplan-Meier curves showing that basal levels of LDH are not predictive of PFS (Log-rank p=NS).
- **Figure 35** shows Kaplan-Meier curves showing that higher basal levels of LDH predict worst OS for melanoma patients (Log-rank p=0.0379).

### EXAMPLE 1: Identification of a miRNA-based non-invasive predictive biomarker of response to target therapy in BRAF-mutant melanoma.

### Material and methods

### Experimental design

Metastatic BRAF-mutated melanoma patients (n = 36 male and n = 34 female) were enrolled in this retrospective study. All of them were treated with the inhibitors of the MAPK pathways, i.e., vemurafenib or dabrafenib (as BRAF inhibitors) alone or in combination with cobimetinib or trametinib respectively (as MEK inhibitors). Serum samples (1ml) of each patient before and after starting MAPK therapy were collected and preserved at the Istituto Nazionale per la Cura dei Tumori "Fondazione G. Pascale", Napoli, Italy or Istituto Nazionale Tumori "Regina Elena" (IFO), Roma, Italy, tumor biobanks.

### Human samples

*The use of human samples was approved by Istituto Pascale's Ethical Committee with the protocol DSC*/*1504 on June 11, 2014 and DSC*/*2893 on April 11, 2015 or by Istituto Nazionale Tumori "Regina Elena" (IFO) Ethical Committee with the protocol* 8393 *of 23.07.2017. All patients signed a general informed consent, which allowed the use of this material for research purposes and which was analyzed in an anonymous manner at the Istituto Nazionale per la Cura dei Tumori "Fondazione G. Pascale" and at Istituto Nazionale Tumori "Regina Elena" (IFO).*

Patients eligible for inclusion in this study were candidates for therapy with BRAF inhibitors alone or in combination with MEK inhibitors. All melanoma patients were ≥ 18 years old and they were able to understand and willing to sign the informed consent form which has been submitted to their attention before any procedure.

The diagnosis was confirmed as locally advanced stage melanoma histology or metastatic lesions according to the American Joint Committee on Cancer - AJCC). The clinical characteristics of patients enrolled in the study such as sex, mutation, therapy, LDH levels refer to patients enrolled at Istituto Nazionale per la Cura dei Tumori "Fondazione G. Pascale" or at Istituto Nazionale Tumori "Regina Elena" (IFO).

### Isolation and evaluation of cfMRNAs

Circulating miRNAs were extracted from the serum of 70 melanoma patients before the beginning of therapy through miRNeasy Mini Kit (Qiagen) according to the manufacturer's instructions. Since the concentration of total RNA was undetectable both using Nanodrop system and Qubit assay, it was arbitrarily decided to reverse transcribe four microliters for each sample as performed in a previous published work [46]. After extraction, expression levels of miRNAs were analyzed using TaqMan MicroRNA assay probes. Real-time PCR for miR-204-5p, miR-199b-5p miR-579-3p, miR-9-5p miR-4443 and miR-4488 was assayed by TaqMan Gene Expression. Data of circulating miRNAs were normalized using both GlobalMean (GMN) normalization and the web-tool RefFinder (RF) (HYPERLINK "http://www.ciidirsinaloa.com.mx/RefFinder-master/" http://www.ciidirsinaloa.com.mx/RefFinder-master/) [49].

To perform GMN, the arithmetic mean of Ct values coming from qRT-PCR analyses has been calculated for each individual sample. This parameter has been then used as a reference value on which all the individual miRNA values have been subtracted to obtain the normalized levels of expression of each miRNA candidate (see below for results).

To perform RF method a web-based comprehensive tool, developed for evaluating and screening reference genes from extensive experimental datasets, has been interrogated. In detail, this tool integrates the results of four different algorithms (i.e., geNorm, Normfinder, BestKeeper, and comparative Delta-Ct method) and allows to identify the most stable biomarker in a pool of a given expression data.

For analysis purposes, ΔCt miRNA values were dichotomized on the basis of the cut-off established using the receiver operating characteristics (ROC) curve considering Progression Disease (PD) specific condition as the state variable. Overall Survival (OS) and Progression Free Survival (PFS) analyses were carried out by the Kaplan-Meier product-limit method. In particular Kaplan-Meier [50] plotter site was used to obtain the graphs (HYPERLINK "https://kmplot.com/analysis/" https://kmplot.com/analysis/). The Log Rank test was used to prove if any statistically significant difference between subgroups exists (p-value < 0.05). In addition the same analysis was conducted taking in exam the Ct raw values of the two miRNAs and their miRatio (*ratio miR-4488* : *miR-579-3p).* All values of AUC, p-*value* and cut-off obtained by GM, RF and Ct are reported in the tables 1, 3 and 4 respectively. Finally, Forest Plots graphs [51] where constructed to compare results of different univariate and bivariate analysis studies related to OS and PFS. The variables used for this analysis are the expression values of the two miRNAs (obtained through GM, RF methods or their raw Ct) and the LDH values.

### Results

### Circulating levels of miR-579-3p and miR-4488 identify BRAF melanoma patients who benefit from target therapy

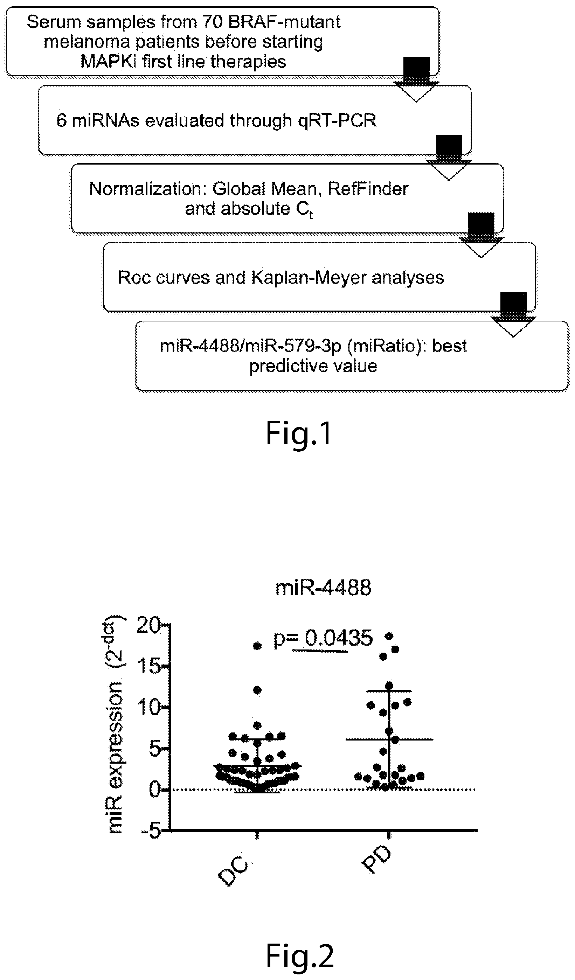

In this retrospective study (Fig.1) serum samples from 70 BRAF-mutant melanoma patients coming from two cancer centers, namely National Cancer Institute IRCCS "G. Pascale Foundation" in Naples and The Regina Elena National Cancer Institute in Rome (IRE), were analyzed. All these advanced melanoma patients were treated with MAPKi (BRAF inhibitors alone or in combination with MEK inhibitors) as first line therapy. Circulating microRNA were extracted from basal serum samples (i.e samples collected before starting therapy) according to procedures described in the material and methods sections. MiRNA levels were analyzed by qRT-PCR. Six miRNAs, previously identified in literature, were analyzed: three oncomiRs, namely miR-9-5p, miR-4443 and miR-4488, and three oncosuppressor miRNAs, i.e. miR-199b-5p, miR-204-5p and miR-579-3p [45,46]. Given that no reference miRNAs are available in the serum, data of circulating miRNAs were normalized using two different methods: GlobalMean Normalization (GMN) [46] and RefFinder, a web-based comprehensive tool developed for evaluating and screening reference genes from extensive experimental datasets. In addition, it was also decided to take in exam the cycle threshold values (Ct values) for the analysis as will be shown subsequently. Data obtained were used to generate ROC and Kaplan-Meier survival curves. The results showed that the ratio between miR-4488 and miR-579-3p levels (miRatio) has the best predictive value of response to MAPKi therapy. An overview of the study is schematically represented in figure 1.

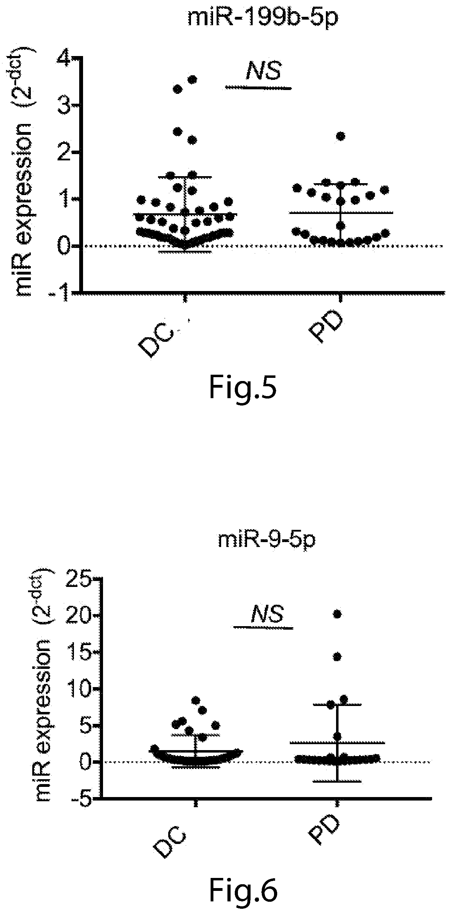

First of all, it was decided to divide the patients according to RECIST criteria in two groups a) those who benefited from treatment with BRAF and MEK inhibitors and b) those who underwent disease progression (PD). In detail, the first group is represented by patients achieving complete response, partial responses or experiencing stable disease (briefly Disease Control or DC) [52,53]. The second group is represented by patients who have not obtained a clinical benefit from the therapy and developed a resistance to MAPKi and have undergone progressive disease (PD). The expression levels of each miRNA was expressed in terms of 2^-dCt values obtained through GMN (Fig. 2-7) [46].

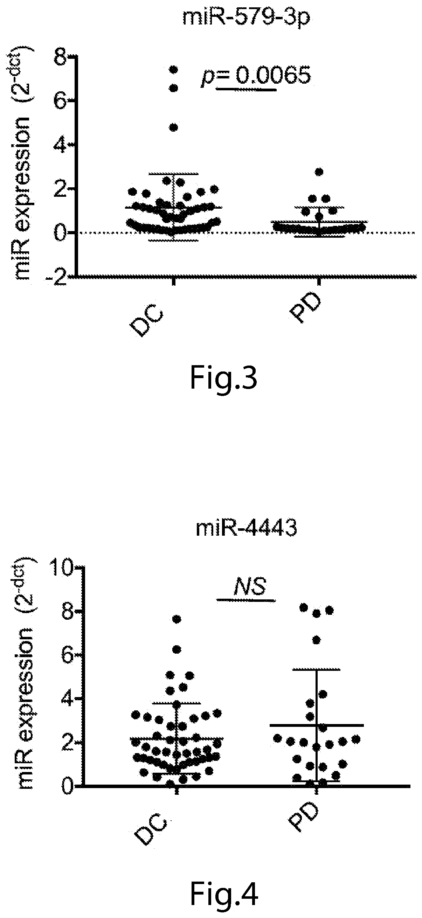

Among the 6 miRNAs analyzed, results revealed that only miR-4488 (Fig.2) and miR-579-3p (Fig.3) showed a significantly different expression (p < 0.05) between the two groups. In particular, melanoma patients undergoing faster PD were characterized by higher circulating levels of miR-4488 as compared to patients experiencing DC. miR-579-3p levels showed the opposite trend. The other miRNAs evaluated resulted not significant in distinguishing PD vs DC patients (Fig.4-7)). On the basis of these results, further studies were focused on these two miRNAs.

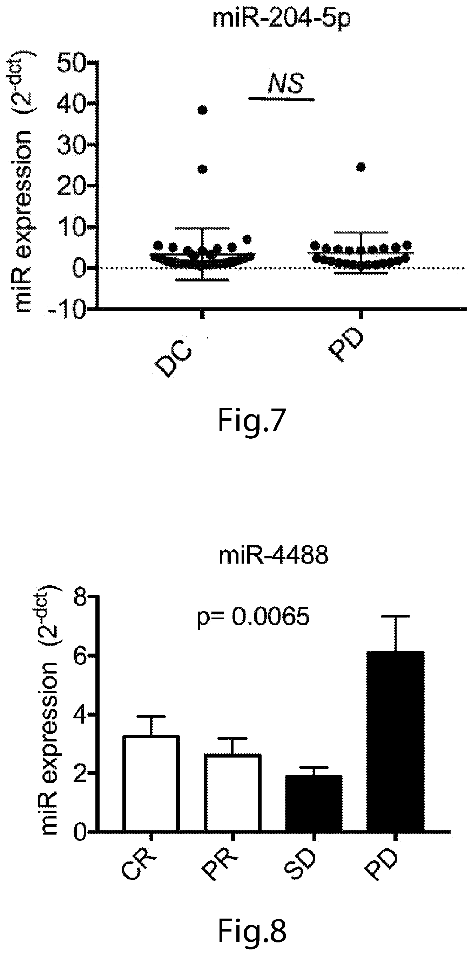

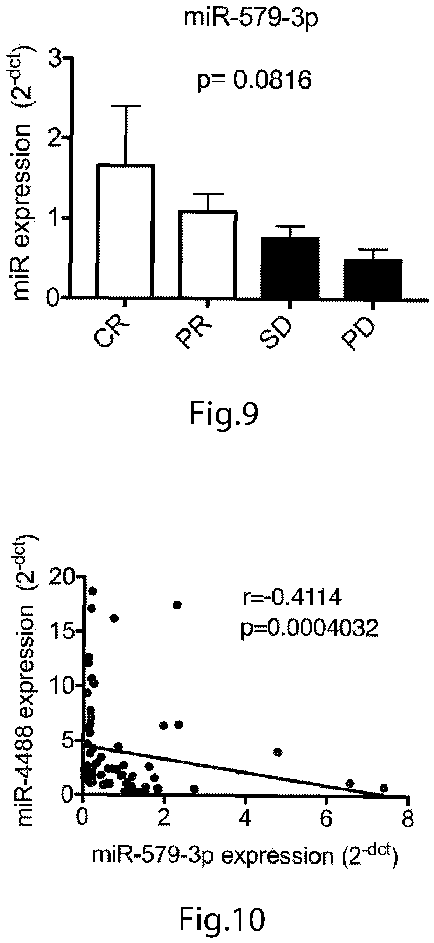

The 70 melanoma patients were further subdivided according to RECIST criteria into the following four groups: CR = complete response; PR = partial response; SD = stable disease; PD = progressive disease; and the distribution of miR-4488 and miR-579-3p was evaluated (Fig.8 and 9). Interestingly it was observed that for miR-4488 the highest expression levels were found in basal serum samples of patients who developed resistance to MAPKi treatment (Fig.8). An opposite trend was observed for miR-579-3p where its expression levels progressively decreased from CR to PD patients (Fig.9). Also, it was observed a significantly negative Spearman correlation of the circulating levels of miR-4488 vs miR-579-3p in basal samples (Fig.10).

Hence, from these data it can be concluded that the circulating levels of the oncosuppressor miR-579-3p and the oncomiR miR-4488 are able to identify, before the beginning of the treatment with MAPKi, BRAF-mutated melanoma patients who will benefit or not from target therapy with BRAF and MEK inhibitors.

### The relative ratio of circulating mir-4488 vs miR-579-3p predicts response to target therapy in BRAF-mutant melanoma patients

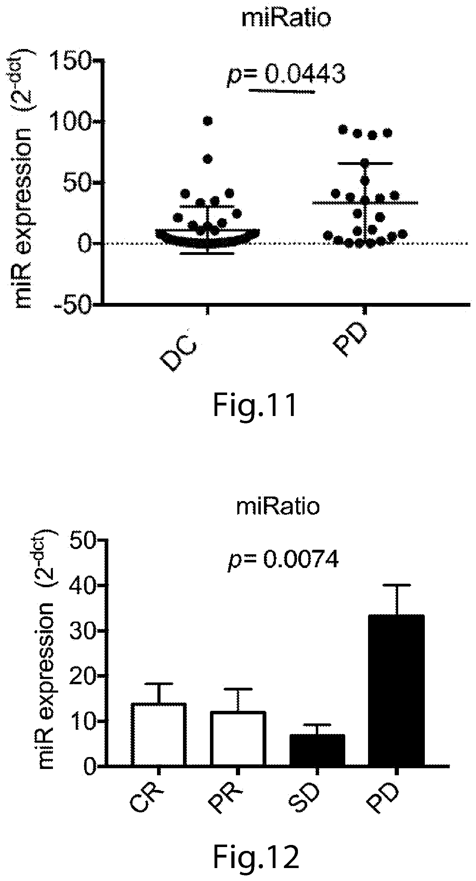

A useful approach to evaluate the expression levels of miRNA in biological fluids is to determine their expression ratio especially when anti-correlated candidates are being taken in exam [42,43]. To this purpose, the relative ratio of the circulating levels of miR-4488 vs miR-579-3p (from here simply miRatio) was calculated based on GMN values. Results clearly showed that miRatio significantly distinguished BRAF-mutated melanoma patients characterized by DC vs PD responses (P < 0.05). In detail, higher levels of this parameter in basal samples deriving from patients who developed faster PD were observed. These data suggest that patients characterized by circulating miR-4488 levels which dominate over miR-579-5p levels had the worst therapeutic response to MAPKi (Fig.11). These findings were confirmed when splitting patients into the four categories according to RECIST criteria, i.e. CR, PR, SD and PD (Fig.12).

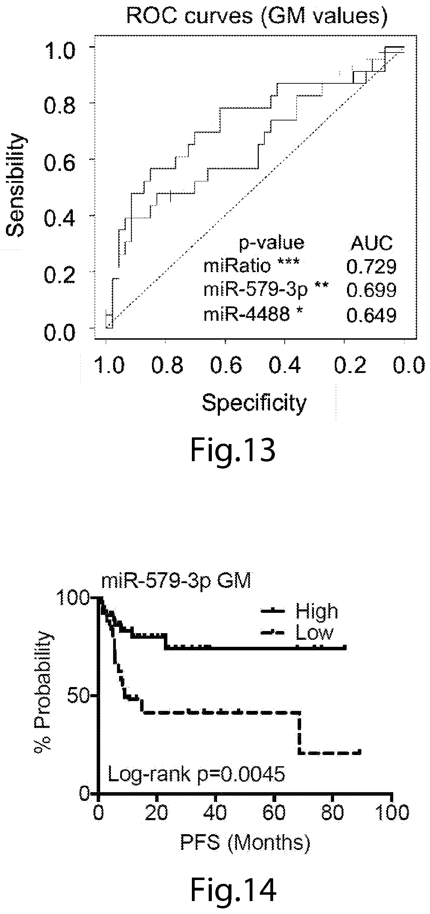

A challenging issue in cancer therapy is the development of tools able to predict patients' response to a given therapy. Hence, the predictive potential of the combination of miR-4488 upregulation and miR-579-3p downregulation was assessed. Their expression levels before therapy start were used to construct receiver operating characteristic (ROC) curves. The parameters of sensitivity, specificity and accuracy were evaluated together in order to generate of the Area Under Curve (AUC) and evaluate the performance of each classifier in a single measure. In detail, miR-579-3p yielded an AUC value of 0.699 whereas miR-4488 of 0.649 (Fig.13) Pleasingly, it was observed that miRatio owns the best predictive value as demonstrated by the highest AUC value, i.e. 0.729, as compared to the individual miRNAs (Fig.13) *(p-value<0.5).*

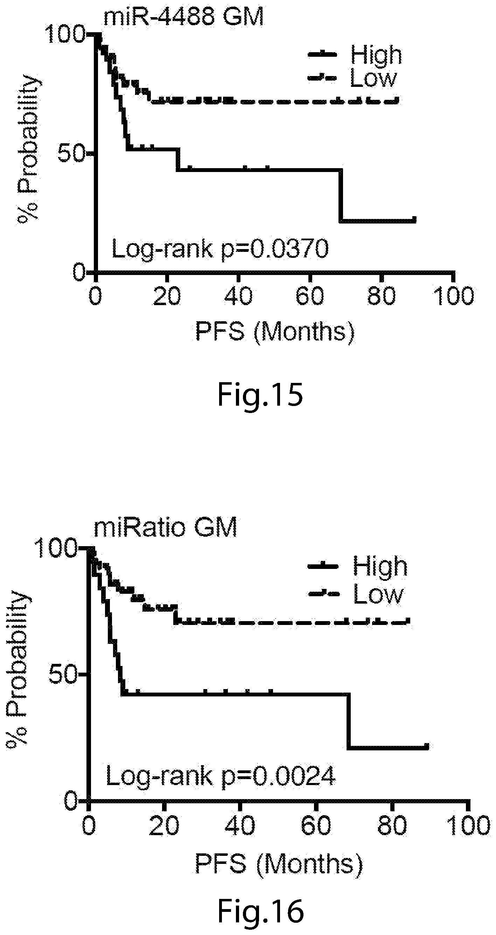

The cut-off calculated through the ROC curves was used to generate the Kaplan-Meier curves in order to estimate whether basal miRNA expression levels may be predictive of Progression-free survival (PFS in months). Results of Kaplan-Meier curve clearly show that high expression levels of miR-579-3p before starting therapy are a predictive factor of better PFS in metastatic melanoma patients as compared to patients with lower levels (Fig.14) of this miRNA. An opposite result was obtained with miR-4488 as shown by Kaplan-Meier curves (Fig.15). Most importantly, the best value to predict PFS in terms of statistical significance (Log-rank p=0.0024) was observed in Kaplan-Meier curves plotted using miRatio values (Fig.16). These results are in line with those of the ROC curves. A summary of values used to generate the graphs are reported in Table 1. In particular, Table 1 reports the AUC, p-values and cut-off values obtained by Global Mean (GM) normalization.

**Table 1**

| **Global mean (GM)** | | | | |
|---|---|---|---|---|
| | **Area Under the Curve (AUC) (95%CI)** | **p-value** | **Test direction** | **Cut-off** |
| **miR-579-3p** | 0.699 (0.567-0.831) | 0.007 | Lower values indicate no response to treatment | 0.2203 |
| **miR-4488** | 0.649 (0.503-0.795) | 0.043 | Higher values indicate no response to treatment | 4.6731 |
| **miRatio** | 0.724 (0.7-0.7) | 0.00094 7328 | Higher values indicate no response to treatment | 24.4484 |

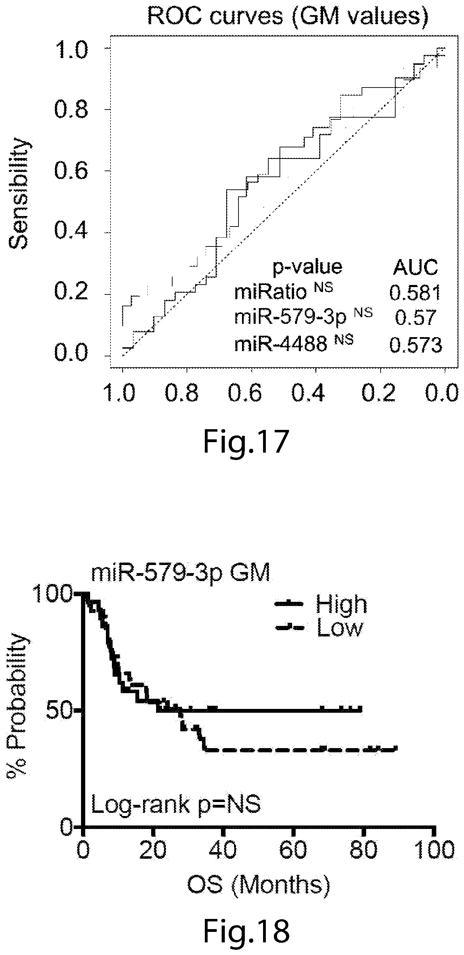

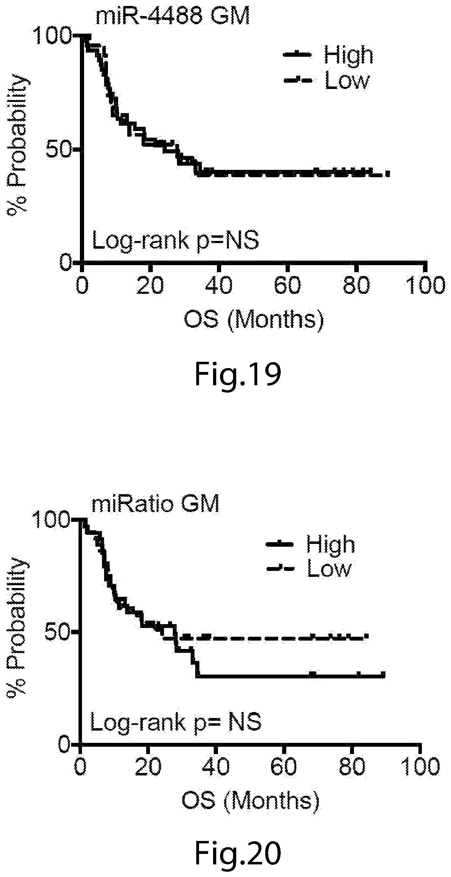

Then it was investigated whether circulating levels of these miRNAs may predict also Overall Survival (OS) for melanoma patients. Again, ROC and Kaplan-Meier curves were plotted using OS as parameter of hypothesis (Fig.17-20). Results revealed that miR-579-3p, miR-4488 and miRatio were not able to predict survival thus suggesting their specificity as a parameter to monitor only PFS.

Finally, to further strengthen these results another normalization method was tested, i.e. the ReFinder normalization, which, as explained above, works by comparing various normalization methods to determine which gene may be the least fluctuating to be used as a reference (Table 2). Exploiting this approach, it has been determined that among the miRNA candidates, miR-199b-5p levels undergoes less fluctuations between the different samples, thereby, it has been used as a reference miRNA. From here the levels of all the other miRNAs candidates (miR-204-5p, miR-579-3p, miR-9-5p miR-4443 and miR-4488) have been normalized on miR-199b-5p expression levels.

Table 2 reports the different normalization results of RefFinder methods and identifies miR-199b-5p as the most stable candidate then used as reference miRNA.

**Table 2**

| | | | | | | |
|---|---|---|---|---|---|---|
| *Delta CT* | miR-204-5p | miR-199b-5p | miR-4443 | miR-579-3p | miR-9-5p | miR-4488 |
| *BestKeeper* | miR-199b-5p | miR-4443 | miR-204-5p | miR-579-3p | miR-9-5p | miR-4488 |
| *NormFinder* | miR-199b-5p | miR-9-5p | miR-4443 | miR-204-5p | miR-579-3p | miR-4488 |
| *Genorm* | miR-199b-5p mir-204-5p | - | miR-4443 | miR-579-3p | miR-9-5p | miR-4488 |
| **Recommended comprehensive ranking** | **miR-199b-5p** | **miR-204-5p** | **miR-4443** | **miR-9-5p** | **miR-579-3p** | **miR-4488** |

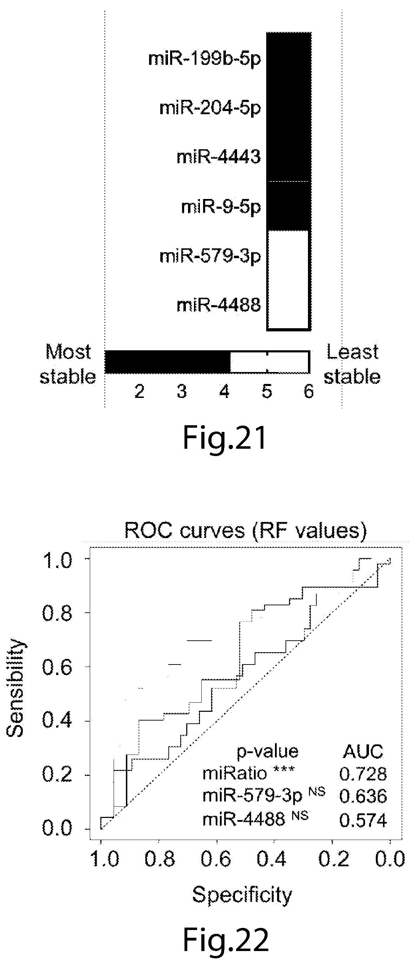

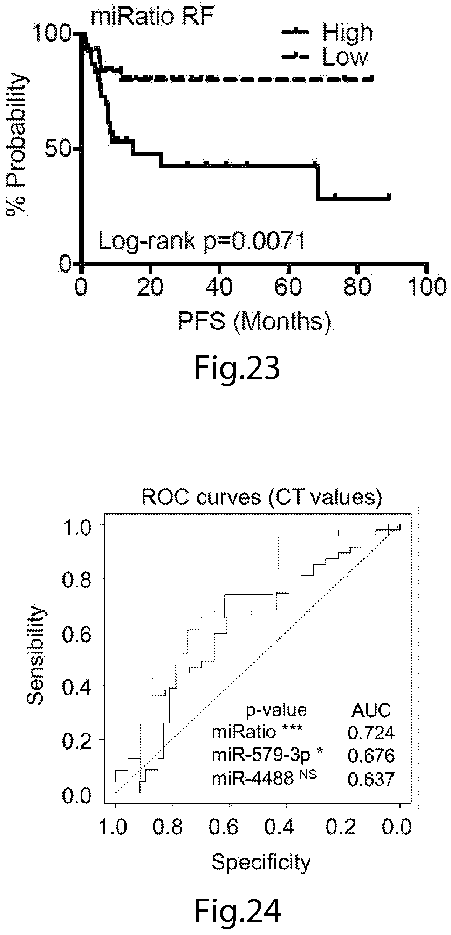

When considering the expression levels of the six miRNAs, it was observed that circulating levels of miR-199b-5p were the most stable among the 70 serum samples tested, therefore becoming the reference miRNA to perform the analyses (Fig.21). Interestingly, ROC curves confirmed also in this case and in agreement with GMN results, that the miRatio yielded the best AUC value (0,728) (Fig.22). These results were used to plot Kaplan-Meier curves, which confirmed that the higher values of miRatio predict a worst PFS as compared to lower levels of this parameter (Fig.23). A summary of values used to generate the graphs by RF method are reported in Table 3, which reports the AUC, p-values and cut-off values obtained by RefFinder (RF) normalization.

**Table 3**

| **Ref Finder (RF)** | | | | |
|---|---|---|---|---|
| | **Area Under the Curve (AUC) (95%Cl)** | **p-value** | **Test direction** | **Cut-off** |
| **miR-579-3p** | 0.636 (0.496-0.775) | 0.067 | Lower values indicate no response to treatment | 0.3768 |
| **miR-4488** | 0.574 (0.429-0.718) | 0.320 | Higher values indicate no response to treatment | 97.5027 |
| **miRatio** | 0.728 (0.7-0.7) | 0.000947328 | Higher values indicate no response to treatment | 7.6459 |

In conclusion, the ratio of miR-4488/miR-579-3p is able to predict response to target therapy in melanoma patients.

### Absolute miRNA expression values are able to predict the development of drug resistance.

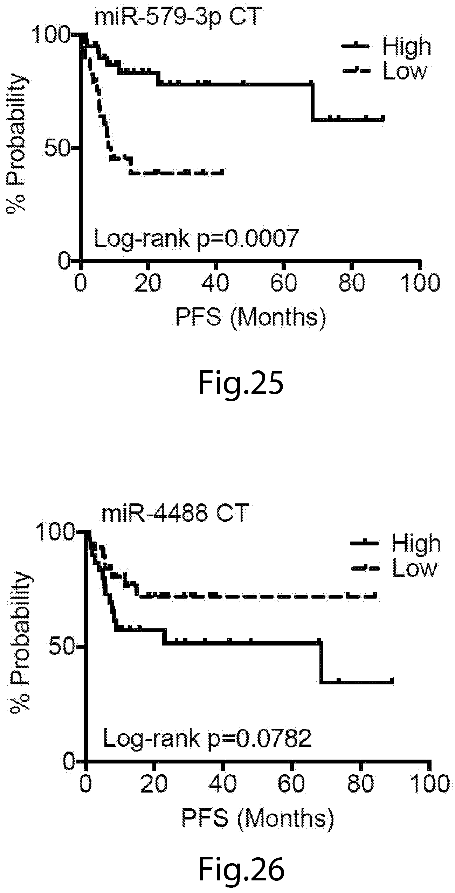

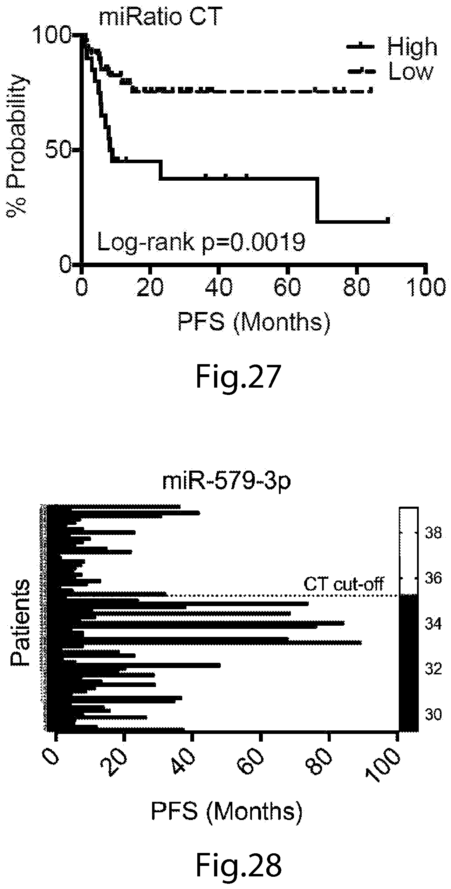

In this part of the study, it was decided to take in exam directly the cycle threshold values (Ct values), i.e. the absolute expression levels of miR-579-3p and miR-4488 as well as their ratio. The goal was to determine whether these parameters may also serve to predict response to target therapy before starting the therapy. This is of interest because in the clinical practice it would be more advantageous to measure absolute expression levels of the miRNA as compared to normalization vs a set of given miRNAs and the results will be of easier interpretation. Data were plotted into ROC curves. As shown in Fig.24, single miRNAs measurements resulted in excellent AUC values. In addition, also in this case the miRatio (based on absolute Ct values) generated a ROC curve with improved AUC value (0,724) and a better significance *(p-value < 0,05).* Once again, the Kaplan-Meier curves plotted using ROC cut-offs confirmed that higher circulating levels of miR-579-3p predicted a better PFS (Fig.25). As expected, higher levels of miR-4488 were associated with worst PFS in melanoma patients (Fig.26). Finally, also the miRNA ratio obtained by using the CT values confirmed the previous findings (Fig.27).

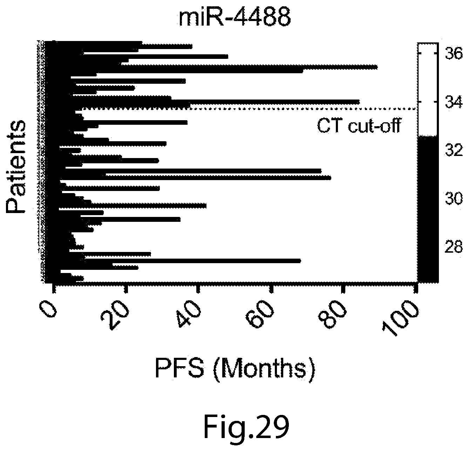

Finally, these results were plotted also as graph bars where melanoma patients were separated by CT cut-off values derived from ROC curves in the groups of high or low expressors of miR-579-3p and miR-4488 respectively. The resulting graphs show that the group of patients with CT cut-off values below median, i.e. the one composed of high miR-579-3p expressors is enriched for longer PFS as compared to the group of patients with CT cut-off values above median (Fig.28). The reverse is true for miR-4488 (Fig.29). Data obtained by CT values are summarized in Table 4, which reports the AUC, p-values and cut-off values obtained by absolute Ct evaluation.

**Table 4**

| **Absolute CT** | | | | |
|---|---|---|---|---|
| | **Area Under the Curve (AUC) (95%CI)** | **p-value** | **Test direction** | **Cut-off** |
| **miR-579-3p** | 0.676 (0.547-0.805) | 0.017 | Higher values indicate no response to treatment | 34.2913 |
| **miR-4488** | 0.637 (0.501-0.774) | 0.063 | Lower values indicate no response to treatment | 30.8466 |
| **miRatio** | 0.724 (0.7-0.7) | 0.000299741 | Higher values indicate no response to treatment | 0.8736 |

### miR-579-3p and miR-4488 are better predictors of PFS as compared to LDH

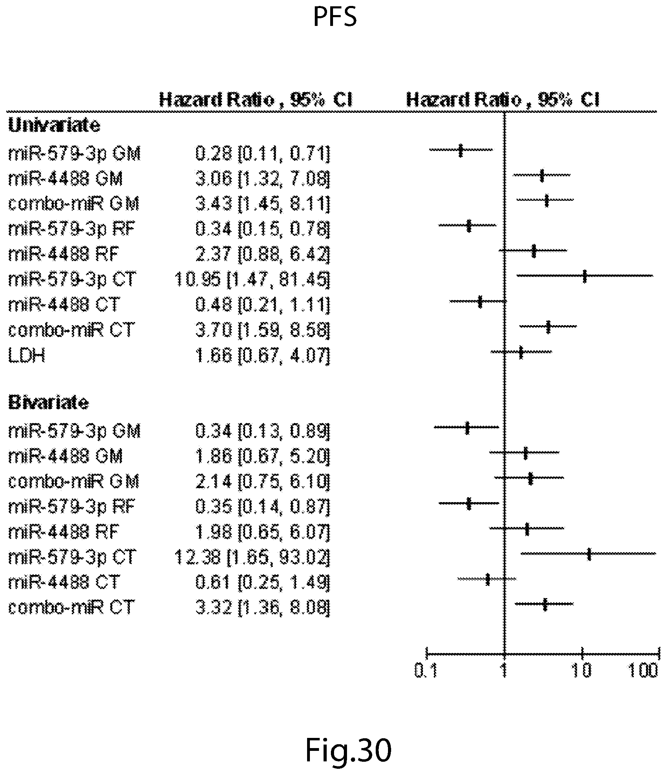

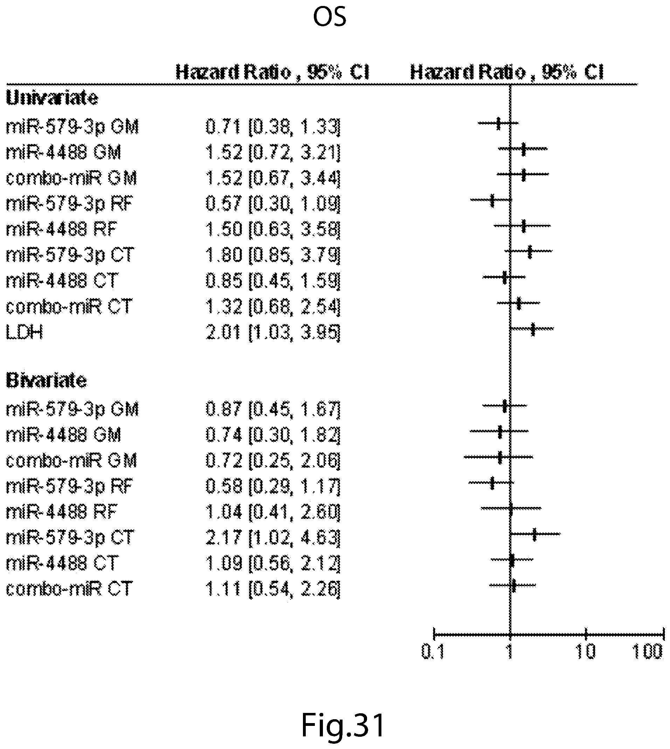

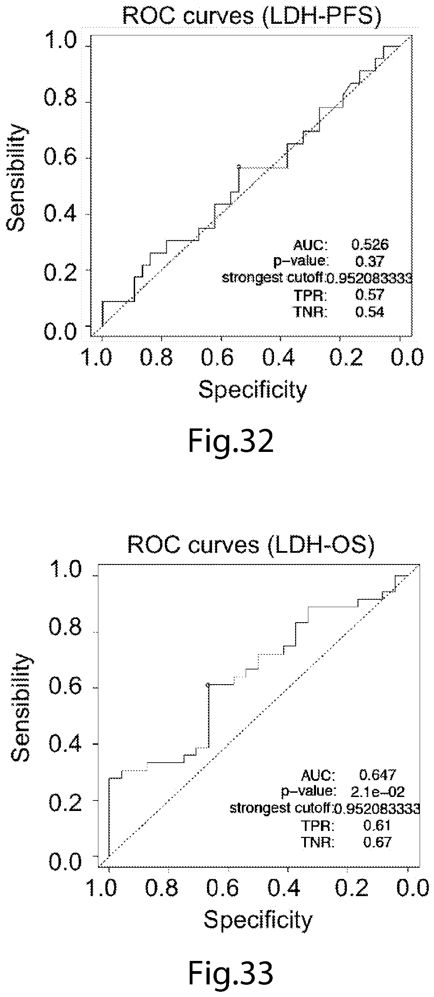

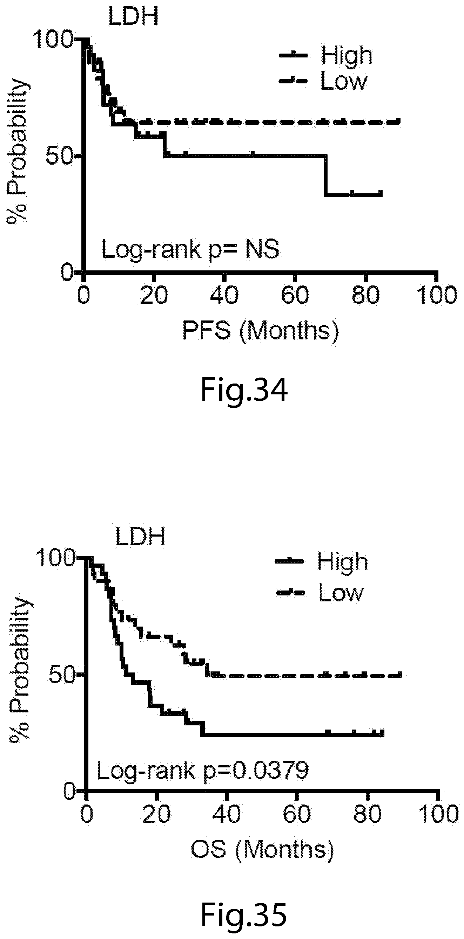

Univariate and multivariate analysis were carried out to assess the predictive value of miRNA levels alone or in combination with the measure of lactate dehydrogenase (LDH) levels in blood. It is well known that baseline LDH value represents one of the main prognostic factors associated with overall survival of patients with advanced melanoma [54,55]. First of all, according to the previous findings, univariate analyses showed a significant effect size of miR-579-3p, miR-4488 and miRatio to predict PFS in all the normalization methods exploited. The Hazard Ratio value (HR) assumes values >1 for oncogenic miR-4488 indicating that its expression is a risk factor in PFS (Fig.30). On the opposite for the oncosuppressor miR-579-3p the HS resulted <1 classifying it as a protective factor (Fig.30). In contrast, LDH was found not to be associated with PFS in univariate analysis as shown by the interval of confidence. Furthermore, considering the multivariate subgroup of analysis, it was observed that the combination of LDH with circulating miRNAs alone or together did not produce any improvement of the HR for predicting PFS (Fig.30). Moreover, Forest Plot graphs showed that the modulation of miR-579-3p and miR-4488 was not associated with OS both in the univariate study and in combination with LDH (bivariate study) (Fig.31). The aspect regarding the association with LDH parameter with PFS was confirmed also by the ROC curve (Fig.32). It is important to point out that, in agreement with other published data [10,55,56], the modulation of LDH alone produced a significative association with the OS. In line with this, ROC curve showed significant AUC value of 0,647 (Fig.33). As expected, the Kaplan-Meier graphs displayed no significative association with PFS parameter (Fig.34) while high levels of LDH in basal serum samples significantly associate with a worst OS (Log-rank p=0.0379). (Fig.35). These data allow to conclude that miR579-3p and miR-4488 are better predictors of PFS as compared to LDH.

### References

1. Russell W J, David E F. Treatment of Advanced Melanoma in 2020 and Beyond. J Invest Dermatol. 2021 Jan;141(1):23-31. doi: 10.1016/j.jid.2020.03.943. Epub 2020 Apr 5.
2. Tanda E T, Vanni I, Boutroa A, et al. Current State of Target Treatment in BRAF Mutated Mela-noma. Front Mol Biosci. 2020 Jul 14;7:154. doi: 10.3389/fmolb.2020.00154. ECollection 2020.
3. Dummer R., Ascierto P.A., Gogas H.J., Arance A., Mandala M., Liszkay G., Garbe C., Schadendorf D., Krajsova I., Gutzmer R., et al. Encorafenib plus binimetinib versus vemurafenib or encorafenib in patients with BRAF-Mutant melanoma (COLUMBUS): A multicentre, open-Label, randomised phase 3 trial. Lancet Oncol. 2018;19:603-615. doi: 10.1016/S1470-2045(18)30142-6.
4. Haas L, Elewaut A, et al. Acquired resistance to anti-MAPK targeted therapy confers an immune-evasive tumor microenvironment and cross-resistance to immunotherapy in melanoma. Nat Can-cer 2, 693-708 (2021). https://doi.org/10.1038/s43018-021-00221-9.
5. Tripathi R, Liu Z, et al. Combating acquired resistance to MAPK inhibitors in melanoma by target-ing Abl1/2-mediated reactivation of MEK/ERK/MYC signaling. Nat Commun. 2020 Oct 29;11(1):5463. doi: 10.1038/s41467-020-19075-3.
6. Ruggiero C F, Malpicci D, et al. ErbB3 Phosphorylation as Central Event in Adaptive Resistance to Targeted Therapy in Metastatic Melanoma: Early Detection in CTCs during Therapy and Insights into Regulation by Autocrine Neuregulin. Cancers (Basel). 2019 Sep 25;11(10):1425. doi: 10.3390/cancers11101425.
7. Pavlick C A, Fecher L, Ascierto P A, Sullivan R J. Frontline Therapy for BRAF-Mutated Metastatic Melanoma: How Do You Choose, and Is There One Correct Answer? Am Soc Clin Oncol Educ Book. 2019 Jan;39:564-571. doi: 10.1200/EDBK_243071. Epub 2019 May 17.
8. Grzywa TM, Paskal W and W 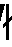 odarski PK. Intratumor and Intertumor Heterogeneity in Melanoma Transl Oncol. 2017 Dec;10(6):956-975. doi: 10.1016/j.tranon.2017.09.007.
9. Hugo W, Shi H, Sun L, et al. Non-genomic and Immune Evolution of Melanoma Acquiring MAP-Ki Resistance Cell. 2015 Sep 10;162(6):1271-85. doi: HYPERLINK "https://dx.doi.org/10.1016%2Fj.cell.2015.07.061" 10.1016/j.cell.2015.07.061
10. Fattore L, Ruggiero C F, et al. The Promise of Liquid Biopsy to Predict Response to Immunother-apy in Metastatic Melanoma. Front Oncol 2021 Mar 18;11:645069. doi: 10.3389/fonc.2021.645069. eCollection 2021.
11. De Rubis G et al. Liquid Biopsies in Cancer Diagnosis, Monitoring, and Prognosis. Trends Phar-macol Sci. 2019 Mar;40(3):172-186. doi: 10.1016/j.tips.2019.01.006. Epub 2019 Feb 5.
12. Chen M, Zhao H. Next-generation sequencing in liquid biopsy: cancer screening and early detec-tion. Hum Genomics 2019; 13: 34. Published online 2019 Aug 1. doi: HYPERLINK "https://dx.doi.org/10.1186%2Fs40246-019-0220-8" 10.1186/s40246-019- HYPERLINK "https://dx.doi.org/10.1186%2Fs40246-019-0220-8" 0220-8.
13. Hayes J, Peruzzi PP, Lawler S. MicroRNAs in cancer: biomarkers, functions and therapy. Trends Mol Med. 2014 Aug 01;2018/07;20 (8):460-469
14. Mumford SL, Towler BP, Pashler AL, et al. Circulating MicroRNA Biomarkers in Melanoma: Tools and Challenges in Personalised Medicine. Biomolecules (2018) 8. 10.3390/biom8020021.
15. Salehi M, Sharifi M. Exosomal miRNAs as novel cancer biomarkers: Challenges and opportuni-ties. J Cell Physiol (2018) 233:6370-80. 10.1002/jcp.26481.
16. Lunavat TR, Cheng L, Einarsdottir BO, et al. BRAFV600 inhibition alters the microRNA cargo in the vesicular secretome of malignant melanoma cells. Proc Natl Acad Sci USA. 2017;114(29):E5930-9
17. Sharon K. Huang, Dave S.B. Hoon. Liquid biopsy utility for the surveillance of cutaneous malig-nant melanoma patients. Mol Oncol. 2016 Mar;10(3):450-63. doi: 10.1016/j.molonc.2015.12.008. Epub 2015 Dec 17.
18. Lim SY, Lee JH, Diefenbach RJ, Kefford RF, Rizos H. Liquid biomarkers in melanoma: detection and discovery. Mol Cancer (2018) 17:8. 10.1186/s12943-018-0757-5
19. Heitzer E. Circulating Tumor DNA for Modern Cancer Management. Clin Chem (2019) 66:143-5. 10.1373/clinchem.2019.304774.
20. Syeda Z A, Langden S S S, et al. Regulatory Mechanism of MicroRNA Expression in Cancer. Int J Mol Sci 2020 Mar 3;21(5):1723. doi: 10.3390/ijms21051723.
21. Acunzo M, Romano G, Wernicke D, et al. MicroRNA and cancer-a brief overview. Adv Biol Regul. 2015;57:1-9.
22. Cui M, Wang H, Yao X, Zhang D, Xie Y, Cui R, et al. Circulating MicroRNAs in Cancer: Poten-tial and Challenge. Front Genet (2019) 10:626. 10.3389/fgene.2019.00626
23. Mumford SL, Towler BP, Pashler AL, et al. Circulating MicroRNA Biomarkers in Melanoma: Tools and Challenges in Personalised Medicine. Biomolecules (2018) 8. 10.3390/biom8020021
24. Condrat C E, Thompson D C et al. miRNAs as Biomarkers in Disease: Latest Findings Regarding Their Role in Diagnosis and Prognosis. Cells. 2020 Feb; 9(2): 276. Published online 2020 Jan 23. doi: HYPERLINK "https://dx.doi.org/10.3390%2Fcells9020276" 10.3390/cells9020276
25. Varrone F, Caputo E. The miRNAs Role in Melanoma and in Its Resistance to Therapy. Int J Mol Sci (2020) 21. 10.3390/ijms21030878
26. Fattore L, Costantini S, Malpicci D, Ruggiero CF, et al. MicroRNAs in melanoma development and resistance to target therapy. Oncotarget (2017) 8:22262-78. 10.18632/oncotarget.14763
27. Fattore L, Sacconi A, Mancini R, Ciliberto G. MicroRNA-driven deregulation of cytokine expres-sion helps development of drug resistance in metastatic melanoma. Cytokine Growth Factor Rev (2017) 36:39-48. 10.1016/j.cytogfr.2017.05.003
28. Salehi M, Sharifi M. Exosomal miRNAs as novel cancer biomarkers: Challenges and opportuni-ties. J Cell Physiol (2018) 233:6370-80. 10.1002/jcp.26481
29. Ge L, Zhang N, et al. Circulating exosomal small RNAs are promising non-invasive diagnostic biomarkers for gastric cancer. J Cell Mol Med 2020 Dec; 24(24): 14502-14513. Published online 2020 Nov 9. doi: HYPERLINK "https://dx.doi.org/10.1111%2Fjcmm.16077" 10.1111/jcmm.16077
30. Wróblewska J P, Lach M S, et al. The Analysis of Inflammation-Related Proteins in a Cargo of Exosomes Derived from the Serum of Uveal Melanoma Patients Reveals Potential Biomarkers of Dis-ease Progression. Cancers (Basel) 2021 Jul; 13(13): 3334. Published online 2021 Jul 2. doi: HYPER-LINK "https://dx.doi.org/10.3390%2Fcancers13133334" 10.3390/cancers13133334
31. Humphries B, Wang Z, et al. MicroRNA Regulation of Breast Cancer Stemness. Int J Mol Sci. 2021 Apr; 22(7): 3756. Published online 2021 Apr 4. doi: HYPERLINK "https://dx.doi.org/10.3390%2Fijms22073756" 10.3390/ijms22073756
32. Wu J, Shen J. Exosomal miRNAs as biomarkers for diagnostic and prognostic in lung cancer. Exo-somal miRNAs as biomarkers for diagnostic and prognostic in lung cancer. Cancer Med 2020 Oct;9(19):6909-6922. doi: 10.1002/cam4.3379. Epub 2020 Aug 10.
33. Daoud A Z, Mulholland E J, et al. MicroRNAs in Pancreatic Cancer: biomarkers, prognostic, and therapeutic modulators. BMC Cancer. 2019 Nov 21;19(1):1130. doi: 10.1186/s12885-019-6284-y.
34. Mirzaei H, Gholamin S, et al. MicroRNAs as potential diagnostic and prognostic biomarkers in melanoma. Eur J Cancer. 2016 Jan;53:25-32. doi: 10.1016/j.ejca.2015.10.009. Epub 2015 Dec 13. ,
35. Ross C L, Kaushik S, et al. MicroRNAs in cutaneous melanoma: Role as diagnostic and prognostic biomarkers. J Cell Physiol. 2018 Jul;233(7):5133-5141. doi: 10.1002/jcp.26395. Epub 2018 Jan 19.
36. Ghafouri-Fard S, Gholipour M, et al. MicroRNA Signature in Melanoma: Biomarkers and Thera-peutic Targets. Front. Oncol., 22 April 2021 | HYPERLINK "https://doi.org/10.3389/fonc.2021.608987"
   https://doi.org/10.3389/fonc.2021.608987
37. Li J, Zhang Z et al. The Diverse Oncogenic and Tumor Suppressor Roles of microRNA-105 in Cancer. Front Oncol. 2019; 9: 518. Published online 2019 Jun 20. doi: HYPERLINK "https://dx.doi.org/10.3389%2Ffonc.2019.00518" 10.3389/fonc.2019.00518.
38. Shao H, Zhang Y, et al. Upregulated MicroRNA-483-3p is an Early Event in Pancreatic Ductal Adenocarcinoma (PDAC) and as a Powerful Liquid Biopsy Biomarker in PDAC. Onco Targets Ther. 2021; 14: 2163-2175. Published online 2021 Mar 25.doi: HYPERLINK "https://dx.doi.org/10.2147%2FOTT.S288936" 10.2147/OTT.S288936
39. Fortunato O, Gasparini P, et al. Exo-miRNAs as a New Tool for Liquid Biopsy in Lung Cancer. Cancers (Basel). 2019 Jun 25;11(6):888. doi: 10.3390/cancers11060888.
40. Margue, C.; Reinsbach, S.; Philippidou, D.; Beaume, N.; Walters, C.; Schneider, J.G.; Nashan, D.; Behrmann, I.; Kreis, S. Comparison of a healthy miRNome with melanoma patient miRNomes: Are microRNAs suitable serum biomarkers for cancer? Oncotarget 2015, 6, 12110-12127
41. Greenberg, E.; Besser, M.J.; Ben-Ami, E.; Shapira-Frommer, R.; Itzhaki, O.; Zikich, D.; Levy, D.; Kubi, A.; Eyal, E.; Onn, A; et al. A comparative analysis of total serum miRNA profiles identifies novel signature that is highly indicative of metastatic melanoma: A pilot study. Biomarkers 2013, 18, 502-508.
42. Boeri M, Verri C, et al. MicroRNA signatures in tissues and plasma predict development and prognosis of computed tomography detected lung cancer. Proc Natl Acad Sci U S A. 2011 Mar 1;108(9):3713-8. doi: 10.1073/pnas.1100048108. Epub 2011 Feb 7.
43. Sun R, Zheng Z, et al. A novel prognostic model based on four circulating miRNA in diffuse large B-cell lymphoma: implications for the roles of MDSC and Th17 cells in lymphoma progression. Mol Oncol. 2021 Jan;15(1):246-261. Doi: 10.1002/1878-0261.12834. Epub 2020 Nov 9.
44. So J B Y, Kapoor R, etv al. Development and validation of a serum microRNA biomarker panel for detecting gastric cancer in a high-risk population. Gut 2021 May;70(5):829-837. doi: 10.1136/gutjnl-2020-322065. Epub 2020 Oct 7.
45. Fattore L, Mancini R, Acunzo M, et al. miR-579-3p controls melanoma progression and resistance to target therapy. Proc Natl Acad Sci USA. 2016;113(34):E5005-13.
46. Fattore L, Ruggiero CF, Pisanu ME et al. Reprogramming miRNAs global expression orchestrates development of drug resistance in BRAF mutated melanoma. Cell Death Differ. 2018 Sep 25. doi: 10.1038/s41418-018-0205-5
47. Fattore L, Campani V, et al. In Vitro Biophysical and Biological Characterization of Lipid Nano-particles Co-Encapsulating Oncosuppressors miR-199b-5p and miR-204-5p as Potentiators of Target Therapy in Metastatic Melanoma. Int J Mol Sci 2020 Mar 12;21(6):1930. doi: 10.3390/ijms21061930.
48. Hong B S, Ryu H S, et al. Tumor Suppressor miRNA-204-5p Regulates Growth, Metastasis, and Immune Microenvironment Remodeling in Breast Cancer. Cancer Res 2019 Apr 1;79(7):1520-1534. doi: 10.1158/0008-5472.CAN-18-0891. Epub 2019 Feb 8.
49. Sarker N, Fabijan J, et al. Identification of stable reference genes for quantitative PCR in koalas. Sci Rep. 2018; 8: 3364. Published online 2018 Feb 20. doi: HYPERLINK "https://dx.doi.org/10.1038%2Fs41598-018-21723-0" 10.1038/s41598-018-21723-0
50. Bhave P, Pallan L, et al. Melanoma recurrence patterns and management after adjuvant targeted therapy: a multicentre analysis. Br J Cancer HYPERLINK "https://www.ncbi.nlm.nih.gov/pmc/articles/PMC7851118/#" . 2021 Feb 2; 124(3): 574-580. Pub-lished online 2020 Oct 22. doi: HYPERLINK "https://dx.doi.org/10.1038%2Fs41416-020-01121-y" 10.1038/s41416-020-01121-y
51. Andrade C. Understanding the Basics of Meta-Analysis and How to Read a Forest Plot: As Simple as It Gets. J Clin Psychiatry 2020 Oct 6;81(5):20f13698. doi: 10.4088/JCP.20f13698.
52. Reschke R, et al. HYPERLINK "https://pubmed.ncbi.nim.nih.gov/30758138/" Rechallenge of targeted therapy in metastatic melanoma. J Dtsch Dermatol Ges 2019 May;17(5):483-486. doi: 10.1111/ddg.13766. Epub 2019 Feb 13.
53. Becco P, Gallo S, et al. Melanoma Brain Metastases in the Era of Target Therapies: An Overview. Cancers (Basel) 2020 Jun 21;12(6):1640. doi: 10.3390/cancers12061640.
54. Deckers E A, Kruijff S, et al. The association between active tumor volume, total lesion glycolysis and levels of S-100B and LDH in stage IV melanoma patients. Eur J Surg Oncol 2020 Nov;46(11):2147-2153. doi: 10.1016/j.ejso.2020.07.011. Epub 2020 Jul 27.
55. Hodi FS, Chiarion-Sileni V, Gonzalez R, Grob J-J, Rutkowski P, Cowey CL, et al.. Nivolumab plus ipilimumab or nivolumab alone versus ipilimumab alone in advanced melanoma (CheckMate 067): 4-year outcomes of a multicentre, randomised, phase 3 trial. Lancet Oncol (2018) 19:1480-92. 10.1016/S1470-2045(18)30700-9
56. Deme D, Telekes A. Prognostic importance of lactate dehydrogenase (LDH) in oncology. Orv He-til . 2017 Dec;158(50):1977-1988. doi: 10.1556/650.2017.30890.

## Claims

1. Method for identifying a non-responsive tumour patient to an anti-tumour treatment with a BRAF inhibitor alone or in combination with a MEK inhibitor, said method comprising
measuring the expression of both microRNAs miR-579-3p and miR-4488 in a biological sample of a tumour patient before starting said anti-tumour treatment,
calculating the ratio between the expression of miR-4488 and the expression of miR-579-3p,
wherein
said tumour patient is a non-responsive tumour patient when said ratio is higher than the ratio between the expression of miR-4488 and the expression of miR-579-3p measured in a biological sample of a responsive tumour patient to the same anti-tumour treatment before starting said anti-tumour treatment.

2. Method according to claim 1, wherein,
when said tumour patient is a non-responsive tumour patient, said ratio is higher than a cut-off ratio value obtained by the following steps:
- measuring the expression of both microRNAs miR-579-3p and miR-4488 in biological samples of a population of tumour patients before starting an anti-tumour treatment with a BRAF inhibitor alone or in combination with a MEK inhibitor, wherein said population of tumour patients comprises a group of non-responsive tumor patients and a group of responsive tumour patients;
- calculating the ratio between the expression of miR-4488 and the expression of miR-579-3p for each tumour patient;
- obtaining a cut-off ratio value through a ROC curve for distinguishing non-responsive tumour patients from responsive tumour patients.

3. Method according to any one of claims 1-2, wherein when said tumour patient is a non-responsive tumour patient
the expression of miR-579-3p measured in the biological sample of said tumour patient is lower than the expression of miR-579-3p measured in a biological sample of a responsive tumour patient to said anti-tumour treatment before starting said anti-tumour treatment
and
the expression of miR-4488 measured in the biological sample of said tumour patient is higher than the expression of miR-4488 measured in a biological sample of a responsive tumour patient to said anti-tumour treatment before starting the anti-tumour treatment.

4. Method according to any one of claims 1-3, wherein the measured expression of miR-579-3p and the measured expression of miR-4488 are determined as absolute levels.

5. Method according to any one of claims 1-3, wherein the measured expression of miR-579-3p and the measured expression of miR-4488 are normalized to the expression of miR-579-3p, miR-4488, miR-204-5p, miR-199b-5p, miR-9-5p and miR-4443.

6. Method according to any one of claims 1-3, wherein the measured expression of miR-579-3p and the measured expression of miR-4488 are normalized to the expression of miR-199b-5p.

7. Method according to any one of claims 1-6, wherein the expression of the miRNAs is measured by means of Real Time PCR, Droplet Digital PCR, Microarray, RNA hybridization methods, such as Northern Blot or Dot Blot, RNA Next Generation Sequencing, preferably Real Time PCR.

8. Method according to any one of claims 1-7, wherein the BRAF inhibitor is selected among Vemurafenib, Dabrafenib, Encorafenib and the MEK inhibitor is selected among and Cobimetinib, Trametinib, Binimetinib.

9. Method according to any one of claims 1-8, wherein the tumour patient is a patient suffering from a BRAF-mutated tumor.

10. Method according to any one of claims 1-9, wherein the tumour patient is a patient suffering from a tumour chosen from the group consisting of melanoma, Colorectal cancer, papillary thyroid carcinoma, non-small cell lung cancer, brain tumour, non-Hodgkin lymphoma.

11. Method according to any one of claims 1-10, wherein the biological sample is a liquid biological sample such as blood, serum, plasma, urine.

12. Method for obtaining a cut-off ratio value for distinguishing non-responsive tumour patients to an anti-tumour treatment with a BRAF inhibitor alone or in combination with a MEK inhibitor from responsive tumour patients to the same treatment, said method comprising
- measuring the expression of both microRNAs miR-579-3p and miR-4488 in biological samples of a population of tumour patients before starting an anti-tumour treatment with a BRAF inhibitor alone or in combination with a MEK inhibitor, wherein said population of tumour patients comprises a group of non-responsive tumour patients and a group of responsive tumour patients;
- calculating the ratio between the expression of miR-4488 and the expression of miR-579-3p for each tumour patient;
- obtaining a cut-off ratio value through a ROC curve.

## Patentansprüche

1. Verfahren zur Identifizierung eines Tumorpatienten, der auf eine Antitumorbehandlung mit einem BRAF-Inhibitor allein oder in Kombination mit einem MEM-Inhibitor nicht anspricht, wobei das Verfahren umfasst
Messung der Expression der beiden microRNAs miR-579-3p und miR-4488 in einer biologischen Probe eines Tumorpatienten vor Beginn der genannten AntiTumorbehandlung,
Berechnung des Verhältnisses zwischen der Expression von miR-4488 und der Expression von miR-579-3p,
wobei
der Tumorpatient ein nicht-ansprechender Tumorpatient ist, wenn das Verhältnis höher ist als das Verhältnis zwischen der Expression von miR-4488 und der Expression von miR-579-3p, gemessen in einer biologischen Probe eines Tumorpatienten, der auf dieselbe Antitumorbehandlung anspricht, bevor die Antitumorbehandlung begonnen wird.

2. Verfahren nach Anspruch 1, wobei,
wenn es sich bei dem Tumorpatienten um einen nicht ansprechenden Tumorpatienten handelt, ist das Verhältnis höher als ein Grenzwert, der durch die folgenden Schritte erhalten wird:
- Messen der Expression der beiden microRNAs miR-579-3p und miR-4488 in biologischen Proben einer Population von Tumorpatienten vor Beginn einer Anti-Tumor-Behandlung mit einem BRAF-Inhibitor allein oder in Kombination mit einem MEK-Inhibitor, wobei die Population von Tumorpatienten eine Gruppe von nichtansprechenden Tumorpatienten und eine Gruppe von ansprechenden Tumorpatienten umfasst;
- Berechnung des Verhältnisses zwischen der Expression von miR-4488 und der Expression von miR-579-3p für jeden Tumorpatienten;
- Ermittlung eines Cut-off-Ratio-Wertes anhand einer ROC-Kurve zur Unterscheidung zwischen nicht ansprechenden Tumorpatienten und ansprechenden Tumorpatienten.

3. Verfahren nach einem der Ansprüche 1-2, wobei, wenn der Tumorpatient ein nicht ansprechender Tumorpatient ist,
die Expression von miR-579-3p, gemessen in der biologischen Probe des Tumorpatienten, niedriger ist als die Expression von miR-579 -3p, gemessen in einer biologischen Probe eines auf die Antitumorbehandlung ansprechenden Tumorpatienten vor Beginn der Antitumorbehandlung,
und
die Expression von miR-4488, gemessen in der biologischen Probe des Tumorpatienten, höher ist als die Expression von miR-4488, gemessen in einer biologischen Probe eines auf die Antitumorbehandlung ansprechenden Tumorpatienten vor Beginn der Antitumorbehandlung.

4. Verfahren nach einem der Ansprüche 1-3, wobei die gemessene Expression von miR-579-3p und die gemessene Expression von miR-4488 als absolute Werte bestimmt werden.

5. Verfahren nach einem der Ansprüche 1-3, wobei die gemessene Expression von miR-579-3p und die gemessene Expression von miR-4488 auf die Expression von miR-579-3p, miR-4488, miR-204-5p, miR-199b-5p, miR-9-5p und miR-4443 normalisiert werden.

6. Verfahren nach einem der Ansprüche 1-3, wobei die gemessene Expression von miR-579-3p und die gemessene Expression von miR-4488 auf die Expression von miR-199b-5p normalisiert werden.

7. Verfahren nach einem der Ansprüche 1-6, wobei die Expression der miRNAs mittels Real Time PCR, Droplet Digital PCR, Microarray, RNA-Hybridisierungsmethoden, wie Northern Blot oder Dot Blot, RNA Next Generation Sequencing, vorzugsweise Real Time PCR, gemessen wird.

8. Verfahren nach einem der Ansprüche 1-7, wobei der BRAF-Inhibitor ausgewählt ist aus Vemurafenib, Dabrafenib, Encorafenib und der MEM-Inhibitor ausgewählt ist aus und Cobimetinib, Trametinib, Binimetinib.

9. Verfahren nach einem der Ansprüche 1-8, wobei der Tumorpatient ein Patient ist, der an einem BRAFmutierten Tumor leidet.

10. Verfahren nach einem der Ansprüche 1-9, wobei der Tumorpatient ein Patient ist, der an einem Tumor leidet, der aus der Gruppe ausgewählt ist, die aus Melanom, kolorektalem Krebs, papillärem Schilddrüsenkarzinom, nicht-kleinzelligem Lungenkrebs, Gehirntumor und Non-Hodgkin-Lymphom besteht.

11. Verfahren nach einem der Ansprüche 1-10, wobei die biologische Probe eine flüssige biologische Probe wie Blut, Serum, Plasma oder Urin ist.

12. Verfahren zur Gewinnung eines Cut-Off-Ratio-Wertes zur Unterscheidung von nicht auf eine Anti-Tumor-Behandlung mit einem BRAF-Inhibitor allein oder in Kombination mit einem MEM-Inhibitor ansprechenden Tumorpatienten von auf dieselbe Behandlung ansprechenden Tumorpatienten, wobei das Verfahren umfasst
- Messen der Expression der beiden microRNAs miR-579-3p und miR-4488 in biologischen Proben einer Population von Tumorpatienten vor Beginn einer Anti-Tumor-Behandlung mit einem BRAF-Inhibitor allein oder in Kombination mit einem MEK-Inhibitor, wobei die Population von Tumorpatienten eine Gruppe von nichtansprechenden Tumorpatienten und eine Gruppe von ansprechenden Tumorpatienten umfasst;
- Berechnung des Verhältnisses zwischen der Expression von miR-4488 und der Expression von miR-579-3p für jeden Tumorpatienten;
- Ermittlung eines Cut-off-Ratio-Wertes anhand einer ROC-Kurve.

## Revendications

1. Procédé d'identification d'un patient atteint d'une tumeur ne répondant pas à un traitement anti-tumoral avec un inhibiteur de BRAF seul ou en combinaison avec un inhibiteur de MEK, ledit procédé comprenant
mesurer l'expression des deux microARN miR-579-3p et miR-4488 dans un échantillon biologique d'un patient atteint d'une tumeur avant de commencer ledit traitement antitumoral,
calculer le rapport entre l'expression de miR-4488 et l'expression de miR-579-3p,
dans lequel
ledit patient tumoral est un patient tumoral non réactif lorsque ledit rapport est supérieur au rapport entre l'expression de miR-4488 et l'expression de miR-579-3p mesuré dans un échantillon biologique d'un patient tumoral réactif au même traitement anti-tumoral avant de commencer ledit traitement anti-tumoral.

2. Procédé selon la revendication 1, dans lequel lorsque le patient atteint d'une tumeur est un patient qui ne réagit pas, ledit rapport est supérieur à une valeur seuil obtenue à l'issue des étapes suivantes:
- mesurer l'expression des deux microARN miR-579-3p et miR-4488 dans des échantillons biologiques d'une population de patients atteints de tumeurs avant de commencer un traitement anti-tumoral avec un inhibiteur de BRAF seul ou en combinaison avec un inhibiteur de MEK, dans lequel ladite population de patients atteints de tumeurs comprend un groupe de patients atteints de tumeurs non réactives et un groupe de patients atteints de tumeurs réactives;
- calculer le rapport entre l'expression de miR-4488 et l'expression de miR-579-3p pour chaque patient atteint d'une tumeur;
- l'obtention d'une valeur seuil de ratio par le biais d'une courbe ROC permettant de distinguer les patients atteints de tumeurs non réactives des patients atteints de tumeurs réactives.

3. Procédé selon l'une des revendications 1-2, dans lequel, lorsque le patient atteint d'une tumeur est un patient atteint d'une tumeur non réactive l'expression de miR-579-3p mesurée dans l'échantillon biologique dudit patient tumoral est inférieure à l'expression de miR-579-3p mesurée dans un échantillon biologique d'un patient tumoral répondant audit traitement antitumoral avant de commencer ledit traitement antitumoral
et
l'expression de miR-4488 mesurée dans l'échantillon biologique dudit patient tumoral est supérieure à l'expression de miR-4488 mesurée dans un échantillon biologique d'un patient tumoral répondant audit traitement antitumoral avant de commencer le traitement antitumoral.

4. Procédé selon l'une des revendications 1-3, dans lequel l'expression mesurée de miR-579-3p et l'expression mesurée de miR-4488 sont déterminées en tant que niveaux absolus.

5. Procédé selon l'une des revendications 1-3, dans lequel l'expression mesurée de miR-579-3p et l'expression mesurée de miR-4488 sont normalisées par rapport à l'expression de miR-579-3p, miR-4488, miR-204-5p, miR-199b-5p, miR-9-5p et miR-4443.

6. Procédé selon l'une des revendications 1-3, dans lequel l'expression mesurée de miR-579-3p et l'expression mesurée de miR-4488 sont normalisées par rapport à l'expression de miR-199b-5p.

7. Procédé selon l'une des revendications 1-6, dans lequel l'expression des miARN est mesurée au moyen d'une PCR en temps réel, d'une PCR numérique en gouttelettes, d'une biopuce, de méthodes d'hybridation de l'ARN, telles que le Northern Blot ou le Dot Blot, du séquençage de l'ARN de nouvelle génération, de préférence au moyen d'une PCR en temps réel.

8. Procédé selon l'une des revendications 1-7, dans lequel l'inhibiteur de BRAF est choisi parmi Vemurafenib, Dabrafenib, Encorafenib et l'inhibiteur de MEK est choisi parmi Cobimetinib, Trametinib, Binimetinib.

9. Procédé selon l'une des revendications 1-8, dans lequel le patient atteint d'une tumeur est un patient souffrant d'une tumeur mutée BRAF.

10. Procédé selon l'une des revendications 1-9, dans lequel le patient atteint d'une tumeur est un patient souffrant d'une tumeur choisie dans le groupe constitué par le mélanome, le cancer colorectal, le carcinome papillaire de la thyroïde, le cancer du poumon non à petites cellules, la tumeur cérébrale, le lymphome non hodgkinien.

11. Procédé selon l'une des revendications 1-10, dans lequel l'échantillon biologique est un échantillon biologique liquide tel que le sang, le sérum, le plasma ou l'urine.

12. Procédé d'obtention d'une valeur de ratio de coupure permettant de distinguer les patients atteints de tumeurs non réactives à un traitement antitumoral par un inhibiteur de BRAF seul ou en combinaison avec un inhibiteur de MEK, des patients atteints de tumeurs réactives au même traitement, ladite méthode comprenant
- mesurer l'expression des deux microARN miR-579-3p et miR-4488 dans des échantillons biologiques d'une population de patients atteints de tumeurs avant de commencer un traitement anti-tumoral avec un inhibiteur de BRAF seul ou en combinaison avec un inhibiteur de MEK, dans lequel ladite population de patients atteints de tumeurs comprend un groupe de patients atteints de tumeurs non réactives et un groupe de patients atteints de tumeurs réactives;
- calculer le rapport entre l'expression de miR-4488 et l'expression de miR-579-3p pour chaque patient atteint d'une tumeur;
- l'obtention d'une valeur de ratio de coupure par le biais d'une courbe ROC.
